(19) Europäisches Patentamt
European Patent Office
Office européen des brevets

(11) EP 0 703 218 A1

(12) EUROPEAN PATENT APPLICATION

(43) Date of publication:
27.03.1996 Bulletin 1996/13

(51) Int Cl.6: **C07D 207/16**, C07D 207/14, C07D 407/06, A61K 31/40

(21) Application number: 95305800.5

(22) Date of filing: 21.08.1995

(84) Designated Contracting States:
AT BE CH DE DK ES FR GB GR IE IT LI LU NL PT SE

(30) Priority: 24.08.1994 US 295337

(71) Applicant: ELI LILLY AND COMPANY
Indianapolis, Indiana 46285 (US)

(72) Inventors:
- Monn, James A.
  Indianapolis, Indiana 46268 (US)
- Tizzano, Joseph P.
  New Palestine, Indiana 46163 (US)
- Valli, Matthew J.
  Indianapolis, Indiana 46208 (US)

(74) Representative: Tapping, Kenneth George et al
Lilly Industries Limited
European Patent Operations
Erl Wood Manor
Windlesham Surrey GU20 6PH (GB)

# (54) Pyrrolidinyl di-carboxylic acid derivatives as metabotropic glutamate receptor antagonists

(57) The present invention provides pyrrolidinyl di-carboxylic acid derivatives that affect certain excitatory amino acid receptors, and are useful in the treatment of neurological disorders and psychiatric disorders. This invention further provides novel pyrrolidinyl di-carboxylic acid derivatives and pharmaceutical formulations employing these novel compounds.

EP 0 703 218 A1

## Description

In the mammalian central nervous system (CNS), the transmission of nerve impulses is controlled by the interaction between a neurotransmitter, that is released by a sending neuron, and a surface receptor on a receiving neuron, which causes excitation of this receiving neuron. L-Glutamate, which is the most abundant neurotransmitter in the CNS, mediates the major excitatory pathway in mammals, and is referred to as an excitatory amino acid (EAA). The receptors that respond to glutamate are called excitatory amino acid receptors (EAA receptors). See Watkins & Evans, Annual Reviews in Pharmacology and Toxicology, 21:165 (1981); Monaghan, Bridges, and Cotman, Annual Reviews in Pharmacology and Toxicology, 29:365 (1989); Watkins, Krogsgaard-Larsen, and Honore, Transactions in Pharmaceutical Science, 11:25 (1990). The excitatory amino acids are of great physiological importance, playing a role in a variety of physiological processes, such as long-term potentiation (learning and memory), the development of synaptic plasticity, motor control, respiration, cardiovascular regulation, and sensory perception.

Excitatory amino acid receptors are classified into two general types. Receptors that are directly coupled to the opening of cation channels in the cell membrane of the neurons are termed "ionotropic." This type of receptor has been subdivided into at least three subtypes, which are defined by the depolarizing actions of the selective agonists *N*-methyl-D-aspartate (NMDA), α-amino-3-hydroxy-5-methylisoxazole-4-propionic acid (AMPA), and kainic acid (KA).

The second general type of receptor is the G-protein or second messenger-linked "metabotropic" excitatory amino acid receptor. This second type is coupled to multiple second messenger systems that lead to enhanced phosphoinositide hydrolysis, activation of phospholipase D, increases or decreases in cAMP formation, and changes in ion channel function. Schoepp and Conn, Trends in Pharmacological Science, 14:13 (1993). Both types of receptors appear not only to mediate normal synaptic transmission along excitatory pathways, but also participate in the modification of synaptic connections during development and throughout life. Schoepp, Bockaert, and Sladeczek, Trends in Pharmacological Science, 11:508 (1990); McDonald and Johnson, Brain Research Reviews, 15:41 (1990).

The excessive or inappropriate stimulation of excitatory amino acid receptors leads to neuronal cell damage or loss by way of a mechanism known as excitotoxicity. This process has been suggested to mediate neuronal degeneration in a variety of conditions. The medical consequences of such neuronal degeneration makes the abatement of these degenerative neurological processes an important therapeutic goal.

The metabotropic glutamate receptors are a highly heterogeneous family of glutamate receptors that are linked to multiple second-messenger pathways. These receptors function to modulate the presynaptic release of glutamate, and the postsynaptic sensitivity of the neuronal cell to glutamate excitation. Agonists and antagonists of these receptors may be useful for the treatment of acute and chronic neurodegenerative conditions, and as antipsychotic, anticonvulsant, analgesic, anxiolytic, antidepressant, and anti-emetic agents.

It is believed that the administration of antagonist compounds, which inhibit the activation of neural receptors, will aid in treating many of the above conditions. Particularly in cases where excitotoxicity mediate the condition, use of an antagonist compound may slow or even halt the process of neuronal cell death.

Antagonists of neural receptors are classified as selective for a particular receptor or receptor subtype, or as non-selective. Antagonists may also be classified as competitive or non-competitive. While competitive and non-competitive antagonists act on the receptors in a different manner to produce similar results, selectivity is based upon the observations that some antagonists exhibit high levels of activity at a single receptor type, and little or no activity at other receptors. In the case of receptor-specific diseases and conditions, the selective antagonists are of the most value.

A well-known selective agonist of metabotropic receptors is (1S,3R)-3-aminocyclopentane-1,3-dicarboxylic acid [ (1S,3R) ACPD]. Other neurotransmitters include L-glutamate, the most abundant in situ neurotransmitter, which stimulates both the ionotropic and metabotropic classes of receptor.

To date there has been no disclosure of an antagonist which is selective for a particular class or subtype of metabotropic glutamate receptor. Selective antagonists for ionotropic receptors have been disclosed, as well as general non-selective antagonists. In order to increase the therapeutic potential for the central nervous system, site-specific, selective antagonists must be developed for each of the different receptor classes and subclasses.

This invention relates to a method of treating or preventing a condition associated with an inappropriate stimulation of a glutamate receptor in a mammal which comprises administering to a mammal in need thereof an effective amount of a compound of Formula I

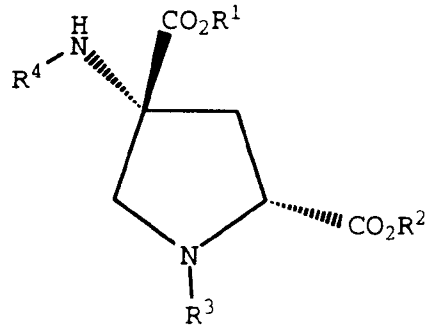

I

wherein:

$R^1$ and $R^2$ are each individually hydrogen or a carboxy protecting group;

$R^4$ is hydrogen or an amino-protecting group; and

$R^3$ is $C_1$-$C_{16}$ alkyl, $C_3$-$C_8$ cycloalkyl, $C_3$-$C_8$ cycloalkenyl, aryl, $C_2$-$C_6$ alkanoyl, $C_2$-$C_6$ alkenoyl, phenyl($C_2$-$C_6$ alkanoyl)-, diphenyl($C_2$-$C_6$ alkanoyl)-, phenyl($C_2$-$C_6$ alkenoyl)-, diphenyl($C_2$-$C_6$ alkenoyl)-, $C_2$-$C_{10}$ alkenyl, $C_2$-$C_{10}$ alkynyl, sulfonyl, aryl ($C_1$-$C_6$ alkylidenyl)-, diaryl($C_1$-$C_6$ alkylidenyl)-, (4-phenyl)phenyl($C_1$-$C_6$ alkylidenyl)-, (4-phenyl)phenyl ($C_2$-$C_6$ alkanoyl)-, heterocyclic, heterocyclic($C_1$-$C_6$ alkylidenyl)-, or a substituted derivative thereof;

or a pharmaceutically acceptable salt or solvate thereof.

The compounds of Formula I are site-specific antagonists of the metabotropic glutamate receptors. In particular, compounds of Formula I have been shown to selectively bind to metabotropic glutamate receptors and to inhibit the phosphoinositide hydrolysis mediated by the known agonist (1S,3R) ACPD.

This invention also provides the novel compounds of Formula Ia

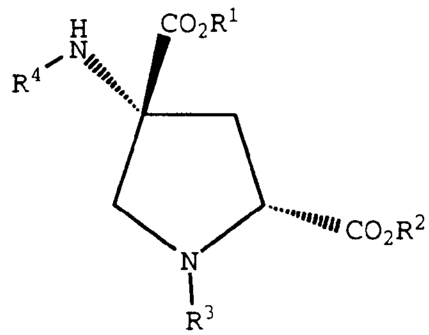

Ia

wherein:

$R^1$ and $R^2$ are each individually hydrogen or a carboxy protecting group;

$R^4$ is hydrogen or an amino-protecting group; and

$R^3$ is $C_1$-$C_{16}$ alkyl, $C_3$-$C_8$ cycloalkyl, $C_3$-$C_8$ cycloalkenyl, aryl, $C_2$-$C_6$ alkanoyl, $C_2$-$C_6$ alkenoyl, phenyl($C_2$-$C_6$ alkanoyl)-, diphenyl ($C_2$-$C_6$ alkanoyl)-, phenyl($C_2$-$C_6$ alkenoyl)-, diphenyl($C_2$-$C_6$ alkenoyl)-, $C_2$-$C_{10}$ alkenyl, $C_2$-$C_{10}$ alkynyl, sulfonyl, aryl($C_1$-$C_6$ alkylidenyl)-, diaryl($C_1$-$C_6$ alkylidenyl)-, (4-phenyl)phenyl($C_1$-$C_6$ alkylidenyl)-, (4-phenyl)phenyl ($C_2$-$C_6$ alkanoyl)-, heterocyclic, heterocyclic($C_1$-$C_6$ alkylidenyl)-, or a substituted derivative thereof;

or a pharmaceutically acceptable salt or solvate thereof.

This invention also provides for pharmaceutical formulations which comprise a compound of Formula Ia in combination with a pharmaceutically acceptable carrier, excipient or diluent.

The terms and abbreviations used in the instant examples have their normal meanings unless otherwise designated. For example "°C" refers to degrees Celsius; "N", refers to normal or normality; "mmol" refers to millimole or millimoles; "g" refers to gram or grams; "ml" means milliliter or milliliters; "M" refers to molar or molarity; "MS" refers to mass spectrometry; "IR" refers to infrared spectroscopy; and "NMR" refers to nuclear magnetic resonance spectroscopy.

The term "$C_1$-$C_{16}$ alkyl" represents a straight or branched alkyl chain having from one to sixteen carbon atoms. Typical straight or branched $C_1$-$C_{16}$ alkyl groups include methyl, ethyl, *n*-propyl, isopropyl, *n*-butyl, isobutyl, sec-butyl, *t*-butyl, *n*-pentyl, isopentyl, *n*-hexyl, 2-methylpentyl, *n*-octyl, decyl and the like. The term "$C_1$-$C_{16}$ alkyl" includes within it the terms "$C_1$-$C_4$ alkyl", "$C_1$-$C_6$ alkyl", and "$C_1$-$C_{10}$ alkyl".

"Halo" represents chloro, fluoro, bromo or iodo.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, t-butoxy, pentoxy and the like.

The term "$C_2$-$C_{12}$ alkenyl" as used herein represents a straight or branched, monovalent, unsaturated aliphatic chain having from two to twelve carbon atoms with at least one double bond. Typical $C_2$-$C_{12}$ alkenyl groups include ethenyl (also known as vinyl), 1-methylethenyl, 1-methyl-l-propenyl, 1-butenyl, 1-hexenyl, 2-methyl-2-propenyl, 1-propenyl, 2-propenyl, 1-butenyl, 2-pentenyl, and the like.

The term "$C_2$-$C_{12}$ alkynyl" refers to a straight or branched, monovalent, unsaturated aliphatic chain having from two to twelve carbon atoms and containing at least one triple bond. Typical $C_2$-$C_{12}$ alkynyl groups include ethynyl, 1-propynyl, 1-butynyl, 1-hexynyl, 2-propynyl, 2-pentynyl, and the like.

The term "$C_3$-$C_8$ cycloalkyl" represents a cyclic alkyl group having three to eight carbon atoms. Typical $C_4$-$C_7$ cycloalkyl groups include cyclobutyl, cyclopentyl, cyclohexyl and cycloheptyl.

The term "$C_2$-$C_6$ alkanoyl" represents a straight or branched alkyl chain having from one to five carbon atoms attached to a carbonyl moiety. Typical $C_2$-$C_6$ alkanoyl groups include acetyl, propanoyl, butanoyl, isobutanoyl, pentanoyl, hexanoyl, 3-methylpentanoyl and the like.

The term "$C_2$-$C_6$ alkenoyl" represents a straight or branched alkenyl chain having from one to five carbon atoms attached to a carbonyl moiety. Typical $C_2$-$C_6$ alkenoyl groups include prop-2-enoyl, but-2-enoyl, but-3-enoyl, isobut-2-enoyl, pentenoyl, hexenoyl, 3-methylpent-2-enoyl and the like.

"$C_1$-$C_{12}$ alkylthio" represents a straight or branched alkyl chain having from one to four carbon atoms attached to a sulfur atom. Typical $C_1$-$C_{12}$ alkylthio groups include methylthio, ethylthio, propylthio, isopropylthio, butylthio and the like. The term "$C_1$-$C_{12}$ alkylthio" includes within its definition the term "$C_1$-$C_6$ alkylthio".

"$C_1$-$C_{12}$ alkylamino" represents a straight or branched alkylamino chain having from one to twelve carbon atoms attached to an amino group. Typical $C_1$-$C_4$ alkylamino groups include methylamino, ethylamino, propylamino, isopropylamino, butylamino, *sec*-butylamino and the like.

The term "$C_2$-$C_7$ alkoxycarbonyl" means a carboxyl group having a $C_1$-$C_6$ alkyl group attached to the carbonyl carbon through an oxygen atom. Representatives of this group include methoxycarbonyl, ethoxycarbonyl, *n*-propoxycarbonyl, *n*-butoxycarbonyl, *t*-butoxycarbonyl, and the like. The preferred alkoxycarbonyl group is methoxycarbonyl.

"$C_3$-$C_8$ cycloalkenyl" represents a hydrocarbon ring structure containing from three to eight carbon atoms and having at least one double bond within that ring.

"Arylsulfonyl" represents an aryl moiety attached through a sulfonyl group.

As would be understood by the skilled artisan, throughout the synthesis of the compounds of Formula I it may be necessary to employ an amino-protecting group or a carboxy-protecting group in order to reversibly preserve a reactively susceptible amino or carboxy functionality while reacting other functional groups on the compound.

Examples of such amino-protecting groups include formyl, trityl, phthalimido, trichloroacetyl, chloroacetyl, bromoacetyl, iodoacetyl, and urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, *t*-butoxycarbonyl, 2-(4-xenyl)-isopropoxycarbonyl, 1,1-diphenyleth-1-yloxycarbonyl, 1,1-diphenylprop-1-yloxycarbonyl, 2-phenylprop-2-yloxycarbonyl, 2-(*p*-toluyl)-prop-2-yloxycarbonyl, cyclopentanyloxy-carbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2-(methylsulfonyl)ethoxycarbonyl, 2-(triphenylphosphino)-ethoxycarbonyl, fluorenylmethoxycarbonyl ("FMOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; benzoylmethylsulfonyl group, 2-nitrophenylsulfenyl, diphenylphosphine oxide and like amino-protecting groups. The species of amino-protecting group employed is not critical so long as the derivatized amino group is stable to the condition of subsequent reaction(s) on other positions of the intermediate molecule and can be selectively removed at the appropriate point without disrupting the remainder of the molecule including any other amino-protecting group(s). Preferred amino-protecting groups are *t*-butoxycarbonyl (*t*-Boc), allyloxycarbonyl and benzyloxycarbonyl (CbZ). Further examples of these groups are found in E. Haslam, PROTECTIVE GROUPS IN ORGANIC CHEMISTRY, (J.G.W. McOmie, ed., 1973), at Chapter 2; and T.W. Greene and P.G.M. Wuts, PROTECTIVE GROUPS IN ORGANIC SYNTHESIS, (1991), at Chapter 7.

Examples of such carboxy-protecting groups include methyl, *p*-nitrobenzyl, *p*-methylbenzyl, *p*-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylene-dioxybenzyl, benzhydryl, 4,4'-dimethoxy-benzhydryl, 2,2',4,4'-tetramethoxybenzhydryl, *t*-butyl, *t*-amyl, trityl, 4-methoxytrityl, 4,4'-dimethoxytrityl, 4,4',4''-trimethoxytrityl, 2-phenylprop-2-yl, trimethylsilyl, *t*-butyldimethylsilyl, phenacyl, 2,2,2-trichloroethyl, β-(di(n-butyl)methylsilyl)ethyl, *p*-toluenesulfonylethyl, 4-nitrobenzylsulfonylethyl, allyl, cinnamyl, 1-(trimethylsilylmethyl)prop-1-en-3-yl and like moieties. Preferred carboxy-protecting groups are allyl, benzyl and *t*-butyl. Further examples of these groups are found in E. Haslam, supra, at Chapter 5; and T.W. Greene and P.G.M. Wuts, supra, at Chapter 5.

The term "aryl" represents an aromatic radical, such as phenyl, and polynuclear aromatic radicals, such as naphthyl, fluorenyl, anthracyl and phenanthrenyl. The term "substituted aryl" represents an aryl group substituted with one or more moieties chosen from the group consisting of halo, hydroxy, cyano, phenyl, nitro, $C_1$-$C_6$ alkyl, $C_1$-$C_6$ alkoxy, carboxy, acetyl, formyl, carboxymethyl, hydroxymethyl, amino, aminomethyl or trifluoromethyl. Examples of substituted aryl groups include 4-methylphenyl, 2-methylphenyl, 4-methoxyphenyl, 4-(*i*-propyl)phenyl, 4-cyclopentylphenyl, 4-(*t*-butyl) phenyl, 4-acetylphenyl, 4-trifluoromethylphenyl, 4-chlorophenyl, 2-bromophenyl, 3-iodophenyl, 6-bromonaphthyl, 3,4-(methylenedioxy)phenyl, indanyl, 1,2,3,4 tetrahydronaphthyl, and 1,2,4,4-tetramethyl-1,2,3,4-tetrahydronaphthyl.

"$C_1$-$C_{10}$ alkylidenyl" refers to a straight or branched, divalent, saturated aliphatic chains of 1 to 10 carbon atoms and includes, but is not limited to, methylenyl, ethylenyl, propylenyl, isopropylenyl, butylenyl, isobutylenyl, *t*-butylenyl, pentylenyl, isopentylenyl, hexylenyl, octylenyl, decylenyl. The term "$C_1$-$C_6$ alkylidenyl" is encompassed within the term "$C_1$-$C_{10}$ alkylidenyl".

The term "aryl($C_1$-$C_6$ alkylidenyl)" represents an aryl group bound through a $C_1$-$C_6$ alkylidenyl group to the core moiety. Representatives of aryl($C_1$-$C_6$ alkylidenyl) groups include benzyl, 1-phenylethyl, 2-phenylethyl, 3-phenylpropyl, 4-phenylbutyl, 2-methyl-2 phenylpropyl, (4-chlorophenyl)methyl, (2,6-dichlorophenyl)methyl, (4-hydroxyphenyl)methyl, (2,4-dinitrophenyl)methyl or the like.

The term "heterocycle" or "heterocyclic" represents a five-membered or six membered ring, which contains one to four heteroatoms selected from oxygen, sulfur and nitrogen. The remaining atoms of the ring are recognized as carbon atoms by those skilled in the art. Rings may be saturated or unsaturated. Examples of heterocycle groups include thiopheneyl, furyl, pyrrolyl, imidazolyl, pyrrazolyl, thiazolyl, thiazolidinyl, isothiazolyl, oxazolyl, isoxazolyl, triazolyl, thiadiazolyl, oxadiazolyl, tetrazolyl, pyridyl, pyrimidyl, pyrazinyl, pyridiazinyl, triazinyl, thiadiazinyl, imidazolyl, dihydropyrimidyl, tetrahydropyrimdyl, pyrrolidinyl, piperidinyl, piperazinyl, pyrazolidinyl, pyrimidinyl, imidazolidimyl, morpholinyl, pyranyl, thiomorpholinyl or the like.

The term "substituted heterocycle" represents a heterocycle group substituted with one or moieties chosen from the group consisting of halo, hydroxy, cyano, nitro, $C_1$-$C_6$ alkyl, $C_1$-$C_4$ alkoxy, alkoxycarbonyl, carboxy, carboxymethyl, hydroxymethyl, amino, aminomethyl or trifluoromethyl. Further, the heterocycle group can be optionally fused to one or two aryl groups to form a benzo-fused group. Examples of a substituted heterocycle include 1,2,3,4-tetrahydrodibenzofuranyl, 2-methylbenzylfuranyl and 3,5 dimethylisoxazolyl.

The term "$C_2$-$C_7$ alkoxycarbonyl" represents a carbonyl group having a $C_1$-$C_6$ alkyl group attached to the carbonyl carbon through an oxygen atom. Examples of this group include *t*-butoxycarbonyl, methoxycarbonyl, and the like.

The term "aryloxycarbonyl" represents a carbonyl group bearing an aryl group attached to the carbonyl carbon through an oxygen atom. Representatives of this group include phenoxycarbonyl, (4-chlorophenoxy) carbonyl, (3-nitrophenoxy)carbonyl, and the like.

This invention provides for compounds which are antagonists of the metabotropic neural receptors in the mammalian central nervous system. The compounds have the general formula:

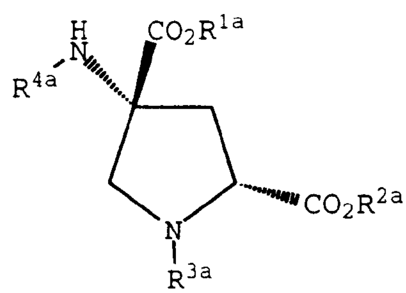

wherein $R^{1a}$, $R^{2a}$ and $R^{4a}$ are each individually hydrogen or a functional protecting group; and

$R^{3a}$ is $C_1$-$C_{16}$ alkyl, $C_3$-$C_8$ cycloalkyl, aryl, $C_1$-$C_6$ alkanoyl, $C_2$-$C_{10}$ alkenyl, $C_2$-$C_{10}$ alkynyl, sulfonyl or any combined derivative of two or more of the above moieties;

or a pharmaceutically acceptable salt or solvate thereof.

As noted, supra, the compounds of the present invention are derivatives of pyrrolidine which are named and numbered according to the RING INDEX, The American Chemical Society, as follows.

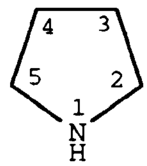

While all of the compounds of Formula I are believed to process antagonist activity at the metabotropic receptors, certain groups of Formula I compounds are more preferred for such use. In this preferred group of compounds, $R^1$, $R^2$ and $R^4$ are all hydrogen atoms which yields N-substituted-4-aminopyrrolidine-2,4-dicarboxylic acids as the preferred group. The preferred methods of this invention are the methods employing those preferred compounds.

These more preferred compounds will be subclassified into three broad categories for purposes of explanation only. The subclassification is based on the structure of the $R^3$ moiety and is in no way limiting of the invention. The $R^3$ groups, for purposes of clarity, will be referred to as the aromatic group; the non-aromatic hydrocarbon group; and the heteroatom group, which includes all $C_1$-$C_6$ alkanoyl and sulfonyl groups. While there will no doubt be some overlap between the third group and the first two, these groupings allow the invention to be described in a precise and concise manner.

As would be expected, the stereochemistry of the Formula I compounds is critical to their potency as antagonist compounds. The relative stereo-chemistry shown in the structures is most preferred with the carboxylic acids preferably in the *trans*-position, and most preferably in the 2R,4S orientation. The 4-amino moiety is preferably *cis* with regard to the 2-carboxy group, in the preferred orientation, 4R.

The relative stereochemistry is preferably established early during synthesis, which avoids stereoisomer separation problems later in the process. Subsequent synthetic step then employ stereospecific procedures so as to maintain the preferred chiralty.

Since the preferred group of compounds includes the same $R^1$, $R^2$, and $R^4$ moieties, the same general scheme of synthesis may be used in producing the preferred compounds, with the only difference occurring in the selection and addition of the $R^3$ moiety.

Scheme I depicted below illustrates the general process used to synthesize the intermediate compound which serves as the backbone for Formula (I) compounds of this invention:

Scheme I

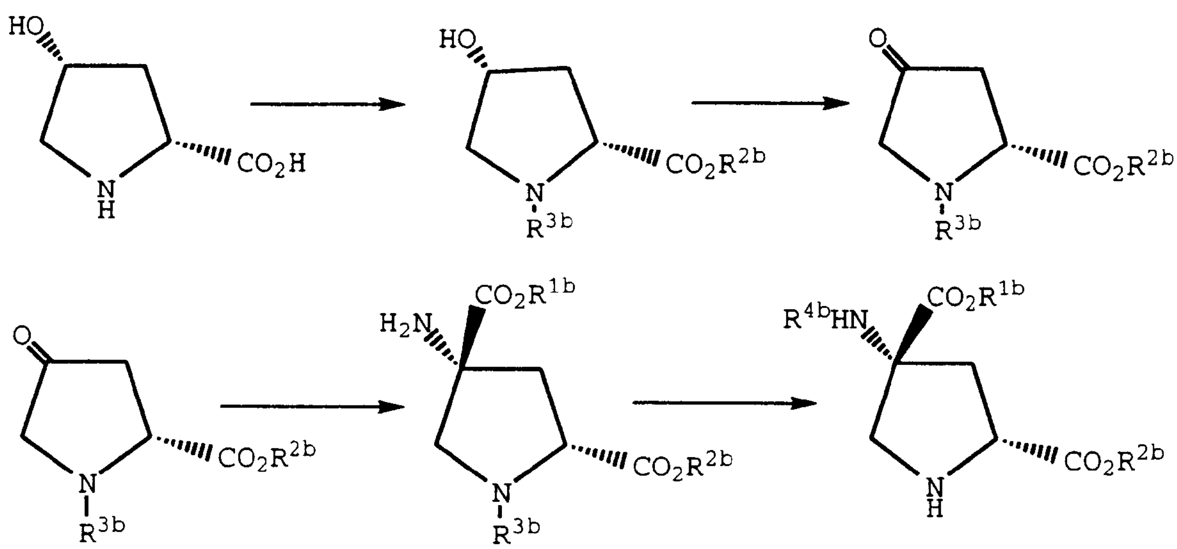

wherein $R^{1b}$ and $R^{2b}$ are carboxy-protecting groups, and R3b is an amino-protecting group.

According to Scheme I, the preferred starting material is *cis*-4-hydroxy-D-proline. Though the series of reactions show, this material is converted into a carboxy-and amino-protected analog of 4-aminopyrrolidine-2,4-dicarboxylic acid. This analog is the backbone from which all of the preferred compounds of Formula I may be synthesized.

As shown in Scheme I, the first step of the synthesis involves the addition of the carboxy protecting group and the addition of a functional group (preferred is an aromatic analog, most preferably benzyl) to the ring nitrogen. The specific reagents and processes for adding protective groups is well-known and will be described in detail in the specific examples infra.

After protection of the 2-carboxy and ring nitrogen, the 4-hydroxy group is oxidized to an oxo group which defines

the cyclic ketone intermediate shown. This intermediate is then disubstituted at the C4 position to add the 4-carboxy and 4-amino moieties. This step generally result in the formation of diasteriomers at the C4 position, which are preferably separated to leave only the desired enantiomer. These 4-substituted groups are protected and the N1 moiety removed to define the final intermediate shown. All specific reagents used and conditions employed in the Scheme I will be identified in the specific examples infra.

The following Schemes II-IV depict the formation of the non-aromatic, aromatic and acylated derivatives of the base intermediate formed by Scheme I.

Scheme II

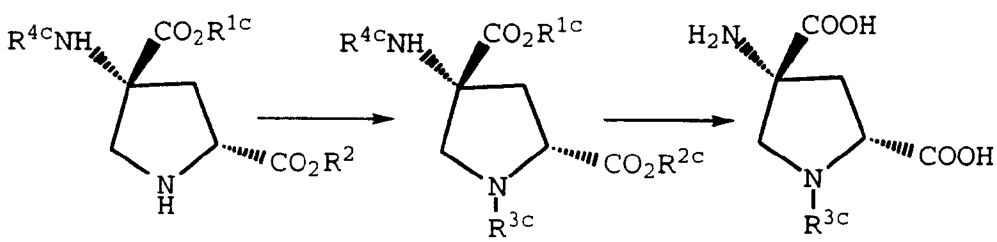

wherein $R^{3c}$ is $C_1$-$C_{10}$ alkyl, $C_2$-$C_{10}$ alkenyl, $C_2$-$C_{10}$ alkynyl or $C_3$-$C_8$ cycloalkyl.

Scheme II depicts the synthesis of the nonaromatic group of preferred compounds of Formula I. This process involves the addition of the desired N1 moiety, normally by a catalyst-assisted reaction of the starting intermediate with an aldehyde of the formula $R^{3c}$-CHO. This intermediate is then deprotected at all three C2-C4-C4 groups to form the desired compound. Deprotection is carried out using standard techniques. Specific reagents and conditions are disclosed in the specific examples later in this specification.

Scheme III

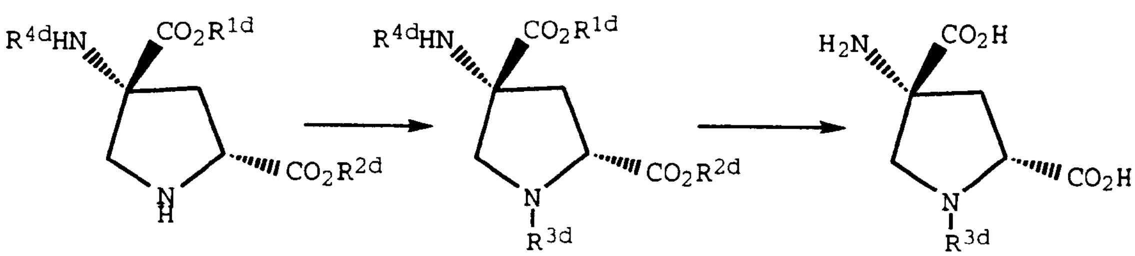

wherein $R^{3d}$ is aryl, aryl($C_1$-$C_6$ alkylidendyl), diaryl, diaryl($C_1$-$C_6$ alkylidenyl)-, (4-phenyl)phenyl, (4-phenyl)phenyl ($C_1$-$C_6$ alkylidendyl), or a substituted derivative thereof.

Scheme III depicts the synthesis of the aromatic group of Formula (I) compounds. This process involves the addition of the N1 aromatic group which is brought about by reacting the starting intermediate with an aromatic halogen in the presence of a tertiary amine. The NI-substituted intermediate is then deprotected as described above to form the N1-aromatic substituted compound shown.

Scheme IV

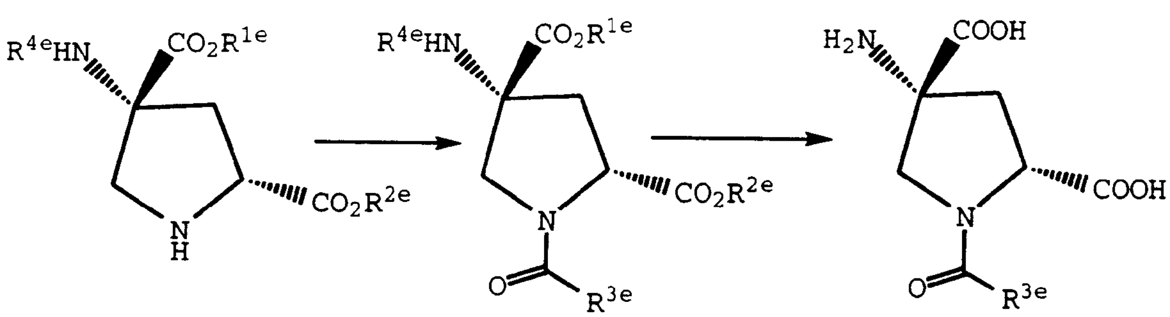

wherein $R^{3e}$ is $C_1$-$C_5$ alkyl, $C_1$-$C_5$ alkenyl, phenyl($C_1$-$C_5$ alkylidenyl)-, diphenyl($C_1$-$C_5$ alkylidenyl)-, phenyl($C_1$-$C_5$

alkenyl)-, (4-phenyl)phenyl($C_1$-$C_5$ alkylidenyl)-, such that when combined with the carbonyl group to which it is attached, forms a $C_2$-$C_6$ alkanoyl, $C_2$-$C_6$ alkenoyl, phenyl($C_2$-$C_6$ alkanoyl)-, diphenyl($C_2$-$C_6$ alkanoyl)-, or phenyl($C_2$-$C_6$ alkenoyl)-, (4-phenyl)phenyl($C_2$-$C_6$ alkanoyl)-, group, respectively, or a substituted derivative thereof.

Scheme IV depicts the synthesis of the N1-acylated group of Formula (I) compounds. One preferred such process involves the addition of the N1-acyl moiety by reacting the starting intermediate with a carbonyl chloride in the presence of a tertiary amine. The resulting intermediate is then deprotected as described above to form the N1-acylated derivative final compound shown.

The most preferred carboxy and nitrogen protecting groups are ethyl and *t*-butyl, respectively. These groups readily bond to their respective functional groups [-C(O)O- and -N<] but do not react appreciably with any of the reagents used in the general synthesis. The protecting groups are easily removed by hydrogenolysis processes well known to those skilled in the art. The general processes of protection and deprotection of certain functional groups has long been accepted in the organic synthesis field.

Each of the three N1-substituted groups includes some compounds which are most preferred for purposes of the methods of this invention. In the aromatic group, the most preferred compounds are 1-benzyl-4-aminopyrrolidine-2,4-dicarboxylic acid; 1-phenethyl-4-aminopyrrolidine-2,4-dicarboxylic acid; 1-methylbenzhydryl-4-aminopyrrolidine-2,4-dicarboxylic acid; 1-(*p*-methoxyphenyl)benzyl-4-aminopyrrolidine-2,4-dicarboxylic acid; 1-(*o*-biphenyl)methyl-4-aminopyrrolidine-2,4-dicarboxylic acid; 1-(*p*-biphenyl)methyl-4-aminopyrrolidine-2,4-dicarboxylic acid; 1-(2-naphthyl)methyl-4-aminopyrrolidine-2,4-dicarboxylic acid; 1-(1-naphthyl)methyl-4-aminopyrrolidine-2,4-dicarboxylic acid; 1-benzhydryl-4-aminopyrrolidine-2,4-dicarboxylic acid; 1-[2(or 3 or 4)-chlorobenzyl]-4-aminopyrrolidine-2,4-dicarboxylic acid; 1-(3,4-diphenoxy)benzyl-4-aminopyrrolidine-2,4-dicarboxylic acid; and 1-(2-phenylsulfonylmethyl)benzyl-4-aminopyrrolidine-2,4-dicarboxylic acid.

Among the most preferred non-aromatic N1-aliphatic compounds are 1-butyl-4-aminopyrrolidine-2,4-dicarboxylic acid; and 1-(*t*-butyl)-4-aminopyrrolidine-2,4-dicarboxylic acid. The most preferred acyl compound is 1-(biphenylmethylcarbonyl)-4-aminopyrrolidine-2,4-dicarboxylic acid.

The following Examples further illustrate the compounds of the present invention and the methods for their synthesis. The Examples are not intended to be limiting to the scope of the invention in any respect, and should not be so construed. All experiments were run under a positive pressure of dry nitrogen or argon. All solvents and reagents were purchased from commercial sources and used as received, unless otherwise indicated. Dry tetrahydrofuran (THF) was obtained by distillation from sodium or sodium benzophenone ketyl prior to use.

Proton nuclear magnetic resonance ($^1$H NMR) spectra were obtained on a GE QE-300 spectrometer at 300.15 MHz, a Bruker AM-500 spectrometer at 500 MHz, or a Bruker AC-200P spectrometer at 200 MHz. Free atom bombardment mass spectroscopy (FABMS) was performed on a VG ZAB-2SE instrument. Field desorption mass spectroscopy (FDMS) was performed using either a VG 70SE or a Varian MAT 731 instrument.

Optical rotations were measured with a Perkin-Elmer 241 polarimeter. Chromatographic separation on a Waters Prep 500 LC was generally carried out using a linear gradient of the solvents indicated in the text unless otherwise specified.

The reactions were generally monitored for completion using thin layer chromatography (TLC). Thin layer chromatography was performed using E. Merck Kieselgel 60 $F_{254}$ plates, 5 cm x 10 cm, 0.25 mm thickness. Spots were detected using a combination of UV and chemical detection (plates dipped in a ceric ammonium molybdate solution [75 g of ammonium molybdate and 4 g of cerium (IV) sulfate in 500 ml of 10% aqueous sulfuric acid] and then heated on a hot plate). Preparative centrifugal thin layer chromatography was performed on a Harrison Model 7924A Chromatotron using Analtech silica gel GF rotors.

Cation exchange chromatography was performed with Dowex® 50X8-100 ion exchange resin. Anion exchange chromatography was performed with Bio-Rad AG® 1-X8 anion-exchange resin (acetate form converted to hydroxide form). Flash chromatography was performed as described by Still, et al., Journal of Organic Chemistry, 43:2923 (1978).

Optical rotations are reported at the sodium-D-line (354 nm). Elemental analyses for carbon, hydrogen, and nitrogen were determined on a Control Equipment Corporation 440 Elemental Analyzer, or were performed by the Universidad Complutense Analytical Centre (Facultad de Farmacia, Madrid, Spain). Melting points were determined in open glass capillaries on a Thomas Hoover capillary melting point apparatus or a Büchi melting point apparatus, and are uncorrected.

Example 1

Synthesis of (2R,4R) diethyl-4-(*tert*-butyloxycarbonylamino)pyrrolidine-2,4-dicarboxylate (scaffold intermediate)

A. Synthesis of (2R,4R) ethyl-1-benzyl-4-hydroxypyrrolidine-2-carboxylate

Concentrated sulfuric acid (15 ml, 267 mmol) was added dropwise to 0°C ethanolic solution (700 ml) of *cis*-4-hydroxy-D-proline (29.75 g, 227 mmol). Upon complete addition, the resulting reaction mixture was refluxed for 10 days with removal of water via a soxhlet condenser filled with 3Å sieves. The reaction mixture was concentrated under reduced pressure and the resulting oil reconstituted in anhydrous methylene chloride (400 ml). N,N-Diisopropylethylamine (77.55 g, 600 mmol) and benzyl bromide (46.6 g, 272 mmol) were consecutively added and the resulting solution stirred at room temperature overnight. Sodium hydroxide (200 ml of a 1N solution) was added to the reaction mixture and the product was extracted with ether (3 x 300 ml). All of the organic phases were combined, washed with brine, dried over potassium carbonate, and concentrated under reduced pressure to afford the crude product, which was purified by high performance liquid chromatography (HPLC) (10% ethyl acetate/ hexanes to 50% ethyl acetate/hexanes) affording the title compound (47.20 g, 189 mmol, 83%). FDMS = 249 ($M^+$). [α]D=+238.81°.

| Analysis for $C_{14}H_{19}NO_3$: | | | |
|---|---|---|---|
| Theory: | C, 67.45; | H, 7.68; | N, 5.26. |
| Found: | C, 67.59; | H, 7.77; | N, 5.68 |

B. Synthesis of (2R) ethyl-1-benzyl-4-oxopyrrolidine-2-carboxylate

Oxalyl chloride (16.0 g, 126 mmol, 11 ml) was added dropwise to a solution of anhydrous methylene chloride (300 ml) and dimethylsulfoxide (13.12 g, 168 mmol) at -78°C. Reaction mixture allowed to equilibrate for 10 minutes, after which time a solution of the 1A intermediate (20.90 g, 84 mmol) in methylene chloride (100 ml) was added dropwise at a rate to keep the reaction temperature below -60°C. Upon complete addition the reaction mixture was allowed to stir at -78°C for 2 hours, then triethylamine (25.50 g, 252 mmol) was added dropwise. After complete addition, the reaction was allowed to warm to room temperature. Water (50 ml) was added to the reaction mixture, the pH was adjusted to 10 with sodium bicarbonate, and the product extracted with ether (3 x 200 ml). All organic phases were combined, washed with brine, dried over potassium carbonate, and concentrated in vacuo to yield crude product which was purified by high performance liquid chromatography (HPLC) (10% ethyl acetate/hexanes to 50% ethyl acetate/ hexanes) affording the title compound (20.44 g, 82.7 mmol) 98%. FDMS =247 $M^+$. $[\alpha]_D$=+44.62°.

| Analysis for $C_{14}H_{17}NO_3$: | | | |
|---|---|---|---|
| Theory: | C, 68.00; | H, 6.93; | N, 5.66. |
| Found: | C, 67.76; | H, 6.91; | N, 5.65. |

C. Synthesis of (2R,4R) diethyl-1-benzyl-4-aminopyrrolidine-2,4-dicarboxylate

Potassium cyanide (13.36 g, 205 mmol) was added in one portion to a solution of the 1B intermediate (20.30 g, 82 mmol) and ammonium carbonate (19.21 g, 246 mmol) in ethanol (500 ml) and water (500 ml). The resulting reaction mixture was heated at 55°C for 2 days. Sodium hydroxide (90.0 g, 2.25 mol) was added and the reaction was warmed under reflux overnight. The reaction mixture was chilled to 0°C, acidified to pH 1 with concentrated hydrochloric acid (~200 ml), and concentrated *in vacuo*. Ethanol (500 ml) was added to the crude amino diacid mixture and then concentrated to dryness (5X), so as to remove residual water. The resulting anhydrous amino diacid was then reconstituted in ethanol (1L), cooled to 0°C, and treated with thionyl chloride (39.02 g, 328 mmol). Upon complete addition the reaction mixture was refluxed for 3 days. The solids were filtered and the filtrate was concentrated in vacuo.

The crude product was partitioned between 3N sodium hydroxide, brine, and ethyl acetate. The ethyl acetate was removed and the aqueous phase extracted with ethyl acetate (3 x 1L). All the organic phases were combined, washed with brine, dried over potassium carbonate, and concentrated in vacuo to yield a dark red oil, which was purified by HPLC (10 % ethyl acetate/hexanes to 90% ethyl acetate/hexanes) affording the title compound (12.14G, 38 mmol) 46%. FDMS =320 (M+). [α] D=203.29.

| Analysis for $C_{17}H_{24}N_2O_4$: | | | |
|---|---|---|---|
| Theory: | C, 63.73; | H, 7.55; | N, 8.74. |
| Found: | C, 63.74; | H, 7.64; | N, 8.50. |

D. Synthesis of (2R,4R) diethyl-1-benzyl-4-(*tert*-butyloxycarbonylamino) pyrrolidine-2,4-dicarboxylate

Di-*tert*-butyl-dicarbonate (12.26 g, 56.2 mmol) was added in one portion to a solution of the 1C intermediate (12.0 g, 37.5 mmol) in methylene chloride (400 ml) and the resulting reaction mixture was stirred at room temperature overnight.

Sodium hydroxide (100 ml of a 0.5 N solution) was added to the reaction mixture and the product was extracted with ether. The organic phases were combined, washed with brine, dried over potassium carbonate, and concentrated in vacuo to yield the crude product, which was purified by HPLC (10% ethyl acetate/hexanes to 50% ethyl acetate/hexanes) affording the title compound (15.92 g, 37.5 mmol), 100% FDMS 420 (M+). [α]D = +99.04°.

| Analysis for $C_{22}H_{32}N_2O_6$: | | | |
|---|---|---|---|
| Theory: | C, 62.84; | H, 7.67; | N, 6.66. |
| Found: | C, 63.06; | H, 7.58; | N, 6.51. |

E. Synthesis of (2R,4R) diethyl-4-(*tert*-butyloxycarbonylamino)pyrrolidine-2,4-dicarboxylate

The intermediate of Example 1D (15.80 g, 37.5 mmol) was added to an ethanolic suspension (100 ml) of 5% palladium on activated carbon catalyst (4.0 g) and exposed to hydrogen gas (60 psi) for 4 hours at room temperature. The reaction mixture was filtered through CELITE® and concentrated in vacuo to yield the crude product, which was purified by HPLC (20% ethyl acetate/hexanes to 80% ethyl acetate/hexanes) affording the title compound (10.48 g, 31.7 mmol) 85%. mp = 58-60°C. FDMS = 331 (M+1). [α]D = +10.63°.

| Analysis for $C_{15}H_{26}N_2O_6$: | | | |
|---|---|---|---|
| Theory: | C, 54.53; | H, 7.93; | N, 8.48. |
| Found: | C, 54.29; | H, 7.79; | N, 8.42. |

Example 2

Synthesis of (2R,4R) diethyl-1(diphenylmethylcarbonyl)-4-(*tert*-butyloxycarbonyl amino)pyrrolidine-2,4-dicarboxylate

Diphenylacetyl chloride (0.43 g, 1.9 mmol) was added dropwise to a solution of (2R,4R) diethyl 4-(*tert*-butyloxycarbonylamino)pyrrolidine-2,4-dicarboxylate (0.50 g, 1.50 mmol) and N,N-diisopropylethylamine (0.50 g, 3.80 mmol) in methylene chloride (30 ml), and the resulting reaction mixture was stirred at room temperature overnight. Hydrochloric acid (25 ml of a 1N solution) was added to the reaction mixture and the product extracted with ether. All organic phases were combined, washed with brine, dried over magnesium sulfate and concentrated *in vacuo* to yield the crude product which was purified by thin layer chromatography (10% ethyl acetate/hexanes to 50% ethyl acetate/hexanes) affording the title compound (0.78 g, 1.50 mmol, 99% yield). [α]D = .

| Analysis for $C_{29}H_{36}N_2O_7$: | | | |
|---|---|---|---|
| Theory: | C, 66.40; | H, 6.92; | N, 5.34. |
| Found: | C, 66.68; | H, 7.00; | N, 5.37. |

Example 3

Synthesis of (2R,4R) 4-amino-1-(benzyl)-pyrrolidine-2,4-dicarboxylic acid

A solution of (2R,4R) diethyl-1-benzyl-4-(tertbutyloxycarbonylamino) pyrrolidine-2,4-dicarboxylate (intermediate of Example 1D) (0.85 g, 2.02 mmol) in a 1:1 mixture of 1 N sodium hydroxide and tetrahydrofuran was stirred at room temperature overnight. The reaction mixture was then concentated to dryness and reconstituted in 6 N hydrochloric acid.

The reaction mixture was then stirred at room temperature to remove the *t*-butoxycarbonyl protecting group. The progress of the reaction was monitored by thin layer chromatography. The reaction mixture was again concentrated to dryness.

The residue was reconstituted in water and applied to a cation exchange column at pH 1.0. The desired product was eluted with 5% pyridine in water to yield 0.51 grams (96%) of the title product as a white solid.

| Analysis for $C_{13}H_{16}N_2O_4$: | | | |
|---|---|---|---|
| Theory: | C, 59.08; | H, 6.10; | N, 10.60. |
| Found: | C, 56.65; | H, 6.05; | N, 10.63. |

Example 4

Synthesis of (2R,4R) diethyl-4-(*tert*-butyloxycarbonylamino)-1-(*n*-pentyl)pyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), 1-pentanal (*n*-valeraldehyde) (0.26 g, 3.0 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(*n*-pentyl)pyrrolidine-2,4-dicarboxylate, was further purified by thin layer chromatography to yield 0.41 g (68%) as a clear oil.

| Analysis. for $C_{20}H_{36}N_2O_6$: | | | |
|---|---|---|---|
| Theory: | C, 59.98; | H, 9.06; | N, 6.99. |
| Found: | C, 59.55; | H, 8.71; | N, 6.68. |

Example 5

Synthesis of (2R,4R) 4-amino-1-(*n*-pentyl)-pyrrolidine-2,4-dicarboxylic acid

The compound of Example 4 (0.35 g, 0.87 mmol) was stirred in diethyl ether at 0°C as anhydrous hydrogen chloride gas was bubbled through the solution until the solution was saturated. The reaction mixture was then permitted to warm to room temperature. The solvents were then removed in vacuo.

The residue was dissolved in tetrahydrofuran (20 ml) and this solution was stirred at room temperature as 1 N sodium hydroxide (20 ml) was added. The resulting reaction mixture was then stirred at room temperature overnight. The solvents were then removed in vacuo.

The residue was reconstituted in water and the pH was adjusted to 2.0. The resulting mixture was then applied to a cation exchange column and the desired product was eluted with 5% pyridine in water. The eluate was then concentrated to dryness, tirturated in water, precipitated with isopropyl alcohol, and filtered to yield 0.23 grams (>99%) of the title product as a white solid.

| Analysis. for $C_{11}H_{20}N_2O_4$: | | | |
|---|---|---|---|
| Theory: | C, 54.08; | H, 8.25; | N, 11.47. |
| Found: | C, 54.34; | H, 8.50; | N, 11.27. |

Example 6

Synthesis of (2R,4R) diethyl-4-(*tert*- butyloxycarbonylamino)-1-(3-phenylpropyl)pyrrolidine-2,4-dicarboxylate

Together were mixed (2R,4R) diethyl-4-(*tert*- butyloxycarbonylamino)pyrrolidine-2,4-dicarboxylate (0.5 g, 1.5 mmol), 3-phenyl-1-propanal (hydrocinnamaldehyde) (0.6 g, 4.5 mmol) and 5% palladium on activated carbon (0.5 g) in ethanol (50 ml) was exposed to hydrogen gas (60 psi) overnight at room temperature. The resulting reaction mixture was then filtred through a CELITE® pad and the desired title product, (2R,4R) diethyl-4-(*tert*-butyloxycarbonylamino)-1-(3-phenylpropyl)pyrrolidine-2,4-dicarboxylate, was further purified by thin layer chromatography to yield 0.87 g (58%) as a clear oil. FDMS 448 (M+)

| Analysis. for $C_{24}H_{36}N_2O_6$: | | | |
|---|---|---|---|
| Theory: | C, 64.26; | H, 8.09; | N, 6.25. |
| Found: | C, 64.24; | H, 8.10; | N, 6.15. |

Example 7

Synthesis of (2R,4R) 4-amino-1-(3-phenylpropyl)pyrrolidine-2,4-dicarboxylic acid

The compound of Example 6 (0.35 g, 0.78 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed

by vacuum.

The residue was reconstituted in water and then applied to a cation exchange column. The desired product was eluted with 5% pyridine in water. The eluate was then concentrated to dryness to yield 0.14 grams (61%) of the title product as a white solid. FDMS 248, 293 (M+1)

| Analysis. for $C_{15}H_{20}N_2O_4$: | | | |
|---|---|---|---|
| Theory: | C, 61.63; | H, 6.90; | N, 9.58. |
| Found: | C, 61.58; | H, 6.50; | N, 8.82. |

Example 8

Synthesis of (2R,4R) diethyl-4-(*tert*-butyloxycarbonylamino)-1-(*neo*-pentyl)pyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), trimethylacetaldehyde (pivalaldehyde) (0.38 g, 4.5 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(*neo*-pentyl)pyrrolidine-2,4-dicarboxylate, was further purified by thin layer chromatography to yield 0.48 g (80%) as a clear oil. FDMS 400 (M+); mp 78-79°C.

| Analysis. for $C_{20}H_{36}N_2O_6$: | | | |
|---|---|---|---|
| Theory: | C, 60.20; | H, 9.19; | N, 6.99. |
| Found: | C, 59.98; | H, 9.06; | N, 6.99. |

Example 9

Synthesis of (2R,4R) 4-amino-1-(*neo*-pentyl)pyrrolidine-2,4-dicarboxylic acid

The compound of Example 8 (0.55 g, 1.37 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and then applied to a cation exchange column. The desired product was eluted with 5% pyridine in water. The eluate was then concentrated to dryness to yield 0.27 grams (81%) of the title product as a white solid. FDMS 200, 245 (M+1)

| Analysis. for $C_{11}H_{20}N_2O_4$: | | | |
|---|---|---|---|
| Theory: | C, 54.08; | H, 8.25; | N, 11.47. |
| Found: | C, 53.79; | H, 8.04 | N, 11.20. |

Example 10

Synthesis of (2R,4R) diethyl-4-(*tert*-butyloxycarbonylamino)-1-propylpyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), propanal (propionaldehyde) (0.26 g, 4.5 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-propylpyrrolidine-2,4-dicarboxylate, was further purified by thin layer chromatography to yield 0.47 g (84%) as a clear oil. FDMS 372 (M+);

| Analysis. for $C_{18}H_{32}N_2O_6$: | | | |
|---|---|---|---|
| Theory: | C, 58.05; | H, 8.66; | N, 7.52. |
| Found: | C, 58.35; | H, 8.46; | N, 7.22. |

Example 11

Synthesis of (2R,4R) 4-amino-1-propylpyrrolidine-2,4-dicarboxylic acid

The compound of Example 10 (0.39 g, 1.04 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and then applied to a cation exchange column. The desired product was eluted with 5% pyridine in water. The eluate was then concentrated to dryness to yield 0.12 grams (53%) of the title product as a white solid. FDMS 172, 217 (M+1)

| Analysis. for $C_9H_{16}N_2O_4$: | | | |
|---|---|---|---|
| Theory: | C, 49.99; | H, 7.46; | N, 12.95. |
| Found: | C, 51.16; | H, 7.62; | N, 13.13. |

Example 12

Synthesis of (2R,4R) diethyl-4-(*tert*-butyloxycarbonylamino)-1-(2,2-diphenylethyl)pyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), diphenylacetaldehyde (0.9 g, 4.5 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(2,2-diphenylethyl)pyrrolidine-2,4-dicarboxylate, was further purified by thin layer chromatography to yield 0.61 g (80%) as a clear oil. FDMS 510 (M+);

| Analysis. for $C_{29}H_{38}N_2O_6$: | | | |
|---|---|---|---|
| Theory: | C, 68.21; | H, 7.50; | N, 5.49. |
| Found: | C, 68.45; | H, 7.50; | N, 5.32. |

Example 13

Synthesis of (2R,4R) 4-amino-1-(2,2-diphenylethyl)pyrrolidine-2,4-dicarboxylic acid

The compound of Example 12 (0.55 g, 1.07 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and the pH was adjusted to 9.0. The mixture was then applied to an anion exchange column. The desired product was eluted with 3 N acetic acid. The eluate was then concentrated to dryness, reconstituted in water, reconcentrated to dryness, reconstituted in water, and then filtered to yield 0.22 grams (58%) of the title product as a white solid. FDMS 172, 217 (M+1)

| Analysis. for $C_{20}H_{22}N_2O_4$: | | | |
|---|---|---|---|
| Theory: | C, 67.78; | H, 6.26; | N, 7.90. |
| Found: | C, 67.54; | H, 6.22; | N, 7.60. |

Example 14

Synthesis of (2R,4R) diethyl-4-(*tert*-butyloxycarbonylamino)-1-butylpyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), 1-butanal (butyraldehyde) (0.32 g, 4.5 mmol) and 5% palladium on activated carbon (0.5 g) in ethanol (50 ml) was exposed to hydrogen gas (60 psi) for overnight at room temperature. The resulting reaction mixture was then filtred

through a CELITE® pad and the desired title product, (2R,4R) diethyl-4-(*tert*-butyloxycarbonylamino)-1-butylpyrrolidine-2,4-dicarboxylate, was further purified by thin layer chromatography to yield 0.36 g (62%) as a clear oil. FDMS 386, 387 (M+);

| Analysis. for $C_{19}H_{34}N_2O_6$: | | | |
|---|---|---|---|
| Theory: | C, 59.05; | H, 8.87; | N, 7.25. |
| Found: | C, 58.92; | H, 8.93; | N, 7.18. |

Example 15

Synthesis of (2R,4R) 4-amino-1-butylpyrrolidine-2,4-dicarboxylic acid

The compound of Example 14 (0.31 g, 0.80 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and the pH was adjusted to 9.0. The mixture was then applied to a cation exchange column. The desired product was eluted with 5% pyridine in water. The eluate was then concentrated to dryness to yield 0.21 grams (>99%) of the title product as a white solid. FDMS 229, 231, 232 (M+1)

| Analysis. for $C_{10}H_{18}N_2O_4$: | | | |
|---|---|---|---|
| Theory: | C, 52.16; | H, 7.88; | N, 12.17. |
| Found: | C, 48.88; | H, 7.86; | N, 11.39. |

Example 16

Synthesis of (2R,4R) diethyl-4-(*tert*-butyloxycarbonylamino)-1-(cyclohexylmethyl)pyrrolidine-2,4-dicarboxylate

Together were mixed (2R,4R) diethyl-4-(*tert*- butyloxycarbonylamino)pyrrolidine-2,4-dicarboxylate (0.5 g, 1.5 mmol), cyclohexanecarboxaldehyde (0.5 g, 4.5 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(cyclohexylmethyl)pyrrolidine-2,4-dicarboxylate, was further purified by thin layer chromatography to yield 0.51g (80%) as a clear oil. FDMS 426 (M+);

| Analysis. for $C_{22}H_{38}N_2O_6$: | | | |
|---|---|---|---|
| Theory: | C, 61.95; | H, 8.98; | N, 6.57. |
| Found: | C, 62.19; | H, 9.12; | N, 6.50. |

Example 17

Synthesis of (2R,4R) 4-amino-1-(cyclohexylmethyl)pyrrolidine-2,4-dicarboxylic acid

The compound of Example 16 (0.47 g, 1.10 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and the pH was adjusted to 9.0. The mixture was then applied to a cation exchange column. The desired product was eluted with 5% pyridine in water. The eluate was then concentrated to dryness to yield 0.27 grams (>91%) of the title product as a white solid. FDMS 271 (M+1)

| Analysis. for $C_{13}H_{22}N_2O_4$: | | | |
|---|---|---|---|
| Theory: | C, 57.76; | H, 8.20; | N, 10.36. |
| Found: | C, 52.76; | H, 7.87; | N, 9.08. |

Example 18

Synthesis of (2R,4R) diethyl-4-(*tert*-butyloxycarbonylamino)-1-(benzylmethyl)pyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), phenylacetaldehyde (0.6 g, 4.5 mmol) and 5% palladium on activated carbon (0.5 g) in ethanol (50 ml) was exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(benzylmethyl)pyrrolidine-2,4-dicarboxylate, was further purified by thin layer chromatography to yield 0.53g (81%) as a light yellow oil. FDMS 434 (M+);

| Analysis. for $C_{23}H_{34}N_2O_6$: | | | |
|---|---|---|---|
| Theory: | C, 63.57; | H, 7.89; | N, 6.45. |
| Found: | C, 63.45; | H, 7.77; | N, 6.25. |

Example 19

Synthesis of (2R,4R) 4-amino-1-(benzylmethyl)pyrrolidine-2,4-dicarboxylic acid

The compound of Example 18 (0.45 g, 1.03 mmol) was stirred in a 1:1 mixture of tetrahydrofuran and sodium hydroxide overnight to saponify the ethyl esters. To the resulting mixture sufficient 6 N hydrochloric acid was added to acidify the mixture to pH 1.0. The resulting mixture was then stirred overnight at room temperature. The solvents were then removed by vacuum.

The residue was reconstituted in water and the pH was adjusted to 9.0. The mixture was then applied to a cation exchange column. The desired product was eluted with 5% pyridine in water. The title product was then recrstallized from water/isopropyl alcohol to yield 0.26 grams (91%) of the title product as a white solid. FDMS **271?** (M+1)

| Analysis. for $C_{14}H_{18}N_2O_4$: | | | |
|---|---|---|---|
| Theory: | C, 60.42; | H, 6.52; | N, 10.07. |
| Found: | C, 58.30; | H, 6.81; | N, 9.66. |

Example 20

Synthesis of (2R,4R) diethyl-4-(*tert*-butyloxycarbonylamino)-1-(4-phenylbutyl)pyrrolidine-2,4-dicarboxylate

Together were mixed (2R,4R) diethyl-4-(*tert*- butyloxycarbonylamino)pyrrolidine-2,4-dicarboxylate (0.5 g, 1.5 mmol), 4-phenylbutanal (0.67 g, 4.5 mmol) and 5% palladium on activated carbon (0.5 g) in ethanol (50 ml). The resulting mixture was then warmed to equilibritate imine formation, and then exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(4-phenylbutyl)pyrrolidine-2,4-dicarboxylate, was further purified by thin layer chromatography to yield 0.38 g (55%) as a light yellow oil. FDMS 462, 463 (M+);

| Analysis. for $C_{25}H_{38}N_2O_6$: | | | |
|---|---|---|---|
| Theory: | C, 64.91; | H, 8.28; | N, 6.06. |
| Found: | C, 64.99; | H, 8.49; | N, 6.01. |

Example 19

Synthesis of (2R,4R) 4-amino-1-(4-phenylbutyl)pyrrolidine-2,4-dicarboxylic acid

The compound of Example 20 (0.34 g, 0.74 mmol) was stirred in a 1:1 mixture of 1 N hydrochloric acid and ethanol (10 ml total volume) overnight. To this was then added 2 ml of concentrated hydrochloric acid and the resulting mxture was then stirred at room temperature for about two days. The solvents were then removed by vacuum.

The residue was reconstituted in 1 N sodium hydroxide (10 ml) and tetrahydrofuran (10 ml) and then stirred overnight at room temperature. Additional sodium hydroxide (5 ml of a 5 N solution) was added to the reaction mixture which was

then stirred overnight at room temperature.

The pH was then adjusted to 10.0 and concentrated to dryness. The residue was then reconstituted in water and applied to an anion exchange column. The desired product was eluted with 50% acetic acid in water to yield 0.19 grams (84%) of the title product as aa off-white solid. FDMS 307 (M+1)

| Analysis. for $C_{16}H_{22}N_2O_4$: | | | |
|---|---|---|---|
| Theory: | C, 62.73; | H, 7.24; | N, 9.14. |
| Found: | C, 58.52; | H, 7.10; | N, 8.53. |

Example 22

Synthesis of (2R,4R) diethyl-4-(*tert*-butyloxycarbonylamino)-1-(5-phenylpentyl)pyrrolidine-2,4-dicarboxylate

Together were mixed (2R,4R) diethyl-4-(*tert*-butyloxycarbonylamino)pyrrolidine-2,4-dicarboxylate (0.5 g, 1.5 mmol), 5-phenylpentanal (0.73 g, 4.5 mmol) and 5% palladium on activated carbon (0.5 g) in ethanol (50 ml). The resulting mixture was then warmed to equilibritate imine formation, and then exposed to hydrogen gas (60 psi) for overnight at room temperature. The resulting reaction mixture was then filtred through a CELITE® pad and the desired title product, (2R,4R) diethyl-4-(*tert*-butyloxycarbonylamino)-1-(5-phenylpentyl)pyrrolidine-2,4-dicarboxylate, was further purified by thin layer chromatography to yield 0.36 g (50%) as a colorless oil. FDMS 476, 477 (M+);

| Analysis. for $C_{26}H_{40}N_2O_6$: | | | |
|---|---|---|---|
| Theory: | C, 65.52; | H, 8.46; | N, 5.88. |
| Found: | C, 67.25; | H, 8.83; | N, 5.33. |

Example 23

Synthesis of (2R,4R) 4-amino-1-(5-phenylpentyl)pyrrolidine-2,4-dicarboxylic acid

The compound of Example 22 (0.30 g, 0.63 mmol) was stirred in a 1:1 mixture of 1 N hydrochloric acid and ethanol (10 ml total volume) overnight. To this was then added 2 ml of concentrated hydrochloric acid and the resulting mixture was then stirred at room temperature for about two days. The solvents were then removed by vacuum.

The residue was reconstituted in 1 N sodium hydroxide (10 ml) and tetrahydrofuran (10 ml) and then stirred overnight at room temperature. Additional sodium hydroxide (5 ml of a 5 N solution) was added to the reaction mixture which was then stirred overnight at room temperature.

The pH was then adjusted to 10.0 and concentrated to dryness. The residue was then reconstituted in water and applied to an anion exchange column. The desired product was eluted with 50% acetic acid in water to yield 0.12 grams (59%) of the title product as a white solid. FDMS 321 (M+1)

| Analysis. for $C_{17}H_{24}N_2O_4$: | | | |
|---|---|---|---|
| Theory: | C, 63.73; | H, 7.55; | N, 8.74. |
| Found: | C, 62.39; | H, 7.98; | N, 7.96. |

Example 24

Synthesis of (2R,4R) diethyl-4-(*tert*-butyloxycarbonylamino)-1-(naphth-2-ylmethyl)pyrrolidine-2,4-dicarboxylate

An amount of (2R,4R) diethyl-4-(*tert*-butyloxycarbonylamino)pyrrolidine-2,4-dicarboxylate (0.75 g, 2.27 mmol) was dissolved in methylene chloride as 2-(bromomethyl)naphthalene (0.75 g, 3.4 mmol) and Hunig's base (N,N-diisopropylethylamine) (0.59 g, 4.54 mmol) were added simultaneously. The resulting mixture was then stirred under a nitrogen atmosphere overnight at room temperature.

The reaction mixture was then partitioned with 1 N sodium hydroxide and the organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.96 g (90%) of the title product as a light yellow oil, which solidified upon standing. FDMS 471 (M+1)

| Analysis for $C_{26}H_{34}N_2O_6$ | | | |
|---|---|---|---|
| Theory: | C, 66.36; | H, 7.28; | N, 5.95. |
| Found: | C, 66.37; | H, 7.46; | N, 5.96. |

Example 25

Synthesis of (2R,4R) 4-amino-1-(naphth-2-ylmethyl)pyrrolidine-2,4-dicarboxylic acid

The compound of Example 24 (0.90 g, 1.91 mmol) was stirred in diethyl ether at 0°C as anhydrous hydrogen chloride was bubbled through the solution. The reaction mixture was then allowed to warm to room temperature at which temperature it was stirred for one hour.

The reaction mixture was then concentrated to dryness and the residue was stirred into a 1:1 mixture of 1 N sodium hydroxide and tetrahydrofuran overnight at room temperature to saponify the esters. The reaction mixture was then neutralized and concentrated to dryness.

The residue was then reconstituted in water, the pH was adjusted to 11, and applied to an anion exchange column. The desired product was eluted with 50% acetic acid in water. The eluate was concentrated to dryness, triturated in a 1:1 mixture of water and methanol, filtered, and washed with acetone and isopropyl alcohol, to yield 0.56 grams (93%) of the title product as a white solid. FDMS 270, 316

| Analysis for $C_{17}H_{18}N_2O_4$: | | | |
|---|---|---|---|
| Theory: | C, 64.96; | H, 5.77; | N, 8.91. |
| Found: | C, 63.35; | H, 5.62 | N, 8.52. |

Example 26

Synthesis of (2R,4R) diethyl-4-(*tert*-butyloxycarbonylamino)-1-(4-phenylbenzyl)pyrrolidine-2,4-dicarboxylate

An amount of (2R,4R) diethyl-4-(*tert*- butyloxycarbonylamino)pyrrolidine-2,4-dicarboxylate (0.75 g, 2.27 mmol) was dissolved in methylene chloride as 4-phenylbenzyl chloride [4-(chloromethyl)biphenyl] (0.69 g, 3.4 mmol) and Hunig's base (N,N-diisopropylethylamine) (0.59 g, 4.54 mmol) were added simultaneously. To the resulting mixture was then added tetrabutylammonium iodide (0.83 g, 2.27 mmol). The resulting mixture was then stirred under a nitrogen atmosphere overnight at room temperature.

The reaction mixture was then partitioned with 1 N sodium hydroxide and the organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 1.05 g (93%) of the title product as a colorless oil. FDMS 497 (M+1)

| Analysis for $C_{28}H_{36}N_2O_6$: | | | |
|---|---|---|---|
| Theory: | C, 67.72; | H, 7.31; | N, 5.64. |
| Found: | C, 67.51; | H, 7.40; | N, 5.41. |

Example 27

Synthesis of (2R,4R) 4-amino-1-(4-phenylbenzyl)pyrrolidine-2,4-dicarboxylic acid

The compound of Example 26 (1.0 g, 2.01 mmol) was stirred in diethyl ether at 0°C as anhydrous hydrogen chloride was bubbled through the solution. The reaction mixture was then allowed to warm to room temperature at which temperature it was stirred for one hour.

The reaction mixture was then concentrated to dryness and the residue was stirred into a 1:1 mixture of 1 N sodium hydroxide and tetrahydrofuran overnight at room temperature to saponify the esters. The reaction mixture was then neutralized and concentrated to dryness.

The residue was then reconstituted in water, the pH was adjusted to 11, and applied to an anion exchange column. The desired product was eluted with 50% acetic acid in water. The eluate was concentrated to dryness, triturated in a 1:1 mixture of water and methanol, filtered, and washed with acetone and isopropyl alcohol, to yield 0.46 grams (68%)

of the title product as a white solid. FDMS 342

| Analysis for $C_{19}H_{20}N_2O_4$: | | | |
|---|---|---|---|
| Theory: | C, 67.05; | H, 5.92; | N, 8.23. |
| Found: | C, 66.85; | H, 5.83; | N, 8.07. |

Example 28

Synthesis of (2R,4R) diethyl-4-(*tert*-butyloxycarbonylamino)-1-(2-phenylbenzyl)pyrrolidine-2,4-dicarboxylate

An amount of (2R,4R) diethyl-4-(*tert*-butyloxycarbonylamino)pyrrolidine-2,4-dicarboxylate (0.75 g, 2.27 mmol) was dissolved in methylene chloride as 2-phenylbenzyl bromide [2-(bromomethyl)biphenyl] (0.84 g, 3.4 mmol) and Hunig's base (N,N-diisopropylethylamine) (0.59 g, 4.54 mmol) were added simultaneously. The resulting mixture was then stirred under a nitrogen atmosphere overnight at room temperature.

The reaction mixture was then partitioned between 1 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 1.11 g (98%) of the title product as a colorless oil. FDMS 497 (M+1)

| Analysis for $C_{28}H_{36}N_2O_6$: | | | |
|---|---|---|---|
| Theory: | C, 67.72; | H, 7.31; | N, 5.64. |
| Found: | C, 67.45; | H, 7.44; | N, 5.57. |

Example 29

Synthesis of (2R,4R) 4-amino-1-(2-phenylbenzyl)pyrrolidine-2,4-dicarboxylic acid

The compound of Example 28 (1.05 g, 2.10 mmol) was stirred in diethyl ether at 0°C as anhydrous hydrogen chloride was bubbled through the solution. The reaction mixture was then allowed to warm to room temperature at which temperature it was stirred for one hour.

The reaction mixture was then concentrated to dryness and the residue was stirred into a 1:1 mixture of 1 N sodium hydroxide and tetrahydrofuran overnight at room temperature to saponify the esters. The reaction mixture was then neutralized and concentrated to dryness.

The residue was then reconstituted in water, the pH was adjusted to 11, and applied to an anion exchange column. The desired product was eluted with 50% acetic acid in water. The eluate was concentrated to dryness, triturated in a 1:1 mixture of water and methanol, filtered, and washed with acetone and isopropyl alcohol, to yield 0.48 grams (67%) of the title product as a white solid. FDMS 296, 341, 342

| Analysis for $C_{19}H_{20}N_2O_4$: | | | |
|---|---|---|---|
| Theory: | C, 67.05; | H, 5.92; | N, 8.23. |
| Found: | C, 67.11; | H, 5.96; | N, 8.16. |

Example 30

Synthesis of (2R,4R) diethyl-4-(*tert*-butyloxycarbonylamino)-1-(4-benzyloxybenzyl)pyrrolidine-2,4-dicarboxylate

An amount of (2R,4R) diethyl-4-(*tert*-butyloxycarbonylamino)pyrrolidine-2,4-dicarboxylate (0.5 g, 1.5 mmol) and Hunig's base (N,N-diisopropylethylamine) (0.40 g, 3.0 mmol) were dissolved in methylene chloride (40 ml) followed by the addition of 4-benzyloxybenzyl bromide (0.84 g, 3.6 mmol). To this reaction mixture was then added tetrabutylammonium iodide (0.55 g, 1.5 mmol). The resulting mixture was then stirred under a nitrogen atmosphere overnight at room temperature.

The reaction mixture was then partitioned with 1 N sodium hydroxide. The organic fraction was removed. The aqueous fraction was extracted with ether, and the extract was combined with the earlier organics. The combined organic fraction was extracted with brine, dried over potassium carbonate, and then concentrated in vacuo. The desired product

was further purified by thin layer chromatography to yield 0.78 g (>99%) of the title product as a light yellow oil. FDMS 526 (M+)

| Analysis for $C_{29}H_{38}N_2O_7$: | | | |
|---|---|---|---|
| Theory: | C, 66.14; | H, 7.27; | N, 5.32. |
| Found: | C, 66.34; | H, 7.03; | N, 5.22. |

Example 31

Synthesis of (2R,4R) 4-amino-1-(4-benzyloxybenzyl)pyrrolidine-2,4-dicarboxylic acid

The compound of Example 30 (0.70 g, 1.30 mmol) was stirred in 1:1 mixture of 1N sodium hydroxide and tetrahydrofuran overnight at room temperature to saponify the ethyl esters. Sufficient 6 N hydrochloric acid was added to acidify the reaction mixture to pH 1.0. The resulting mixture was then stirred overnight at room temperature.

The reaction mixture was then concentrated to dryness and the residue was stirred into a 1:1 mixture of water and methanol overnight at room temperature to saponify the esters. The reaction mixture was then filtered and and the filtrate was applied to a cation exchange column, eluting with 5% pyridine in water. The solvents were then removed by vacuum.

The solid removed by filtration was washed with isopropyl alcohol and then with acetone. All the solids were combined, basified to pH 14 with 1 N sodium hydroxide, and then concentrated to dryness. The residue was reconsitituted in water, acidified to pH 9.0, and then applied to an anion exchange resin. The desired product was eluted with 3 N acetic acid, the eluate being concentrated to dryness. The residue was reconstituted in water and then concentrated to dryness. The residue was then reconstituted in hot water and then filtered. The solids were washed with isopropyl followed by diethyl ether to yield 0.29 grams (60%) of the desired product as a white solid. FDMS 371 (M+1).

| Analysis for $C_{20}H_{22}N_2O_5$: | | | |
|---|---|---|---|
| Theory: | C, 64.85; | H, 5.99; | N, 7.56. |
| Found: | C, 68.65; | H, 5.84; | N, 6.66. |

Example 32

Synthesis of (2R,4R) diethyl-4-(*tert*-butyloxycarbonylamino)-1-(2-iodobenzyl)pyrrolidine-2,4-dicarboxylate

An amount of (2R,4R) diethyl-4-(*tert*-butyloxycarbonylamino)pyrrolidine-2,4-dicarboxylate (1.0 g, 3.03 mmol), Hunig's base (N,N-diisopropylethylamine) (0.78 g, 6.06 mmol), 2-iodobenzyl chloride (0.92 g, 3.64 mmol), and tetrabutylammonium iodide (1.12 g, 3.03 mmol) were dissolved in methylene chloride (25 ml). The resulting mixture was then stirred under a nitrogen atmosphere at room temperature for five days.

The reaction mixture was then partitioned between 0.5 N sodium hydroxide and methylene chloride. The organic fraction was removed. The aqueous fraction was extracted with ether, and the extract was combined with the earlier organics. The combined organic fraction was washed with water, followed by a wash with brine, dried over potassium carbonate, and then concentrated in vacuo. The desired product was further purified by thin layer chromatography to yield 1.55 g (94%) of the title product as a colorless oil. FDMS 546 (M+)

| Analysis for $C_{22}H_{31}IN_2O_6$: | | | |
|---|---|---|---|
| Theory: | C, 48.36; | H, 5.72; | N, 5.13. |
| Found: | C, 48.48; | H, 5.67; | N, 4.86. |

Example 33

Synthesis of (2R,4R) 4-amino-1-(2-iodobenzyl)pyrrolidine-2,4-dicarboxylic acid

The compound of Example 32 (0.42 g, 0.77 mmol) was stirred in ethyl acetate chilled to 0°C. Anhydrous hydrogen chloride was then bubbled through the solution until saturated.

The reaction mixture warmed to room temperature and then stirred at room temperature for about three hours. The reaction mixture was concentrated to dryness, reconstituted in tetrahydrofuran (15 ml) and 1 N sodium hydroxide (15 ml), and then stirred at room temperature overnight.

The tetrahydrofuran was removed in vacuo, and the aqueous solution was adjusted to pH 10, and then applied to an anion exchange resin, eluting with 3 N acetic acid. The desiredtitle product was recrystallized from water to yield 0.24 grams (80%) as a white solid. FDMS 391 (M+1).

| Analysis for $C_{13}H_{15}IN_2O_4$: | | | |
|---|---|---|---|
| Theory: | C, 40.02; | H, 3.87; | N, 7.18. |
| Found: | C, 38.85; | H, 3.85; | N, 6.51. |

Example 34

Synthesis of (2R,4R) diethyl-4-(*tert*-butyloxycarbonylamino)-1-(4-dodecylbenzyl)pyrrolidine-2,4-dicarboxylate

An amount of (2R,4R) diethyl-4-(*tert*-butyloxycarbonylamino)pyrrolidine-2,4-dicarboxylate (0.35 g, 1.06 mmol), Hunig's base (N,N-diisopropylethylamine) (0.27 g, 2.12 mmol), 4-dodecylbenzyl chloride (0.36 g, 1.27 mmol), and tetrabutylammonium iodide (0.40 g, 1.06 mmol) were dissolved in methylene chloride (25 ml). The resulting mixture was then stirred under a nitrogen atmosphere at room temperature for seven days.

The reaction mixture was then partitioned between 0.5 N sodium hydroxide and methylene chloride. The organic fraction was removed. The aqueous fraction was extracted with ether, and the extract was combined with the earlier organics. The combined organic fraction was washed with water, followed by a wash with brine, dried over potassium carbonate, and then concentrated in vacuo. The desired product was further purified by thin layer chromatography to yield 0.48 g (77%) of the title product as a colorless oil. FDMS 588 (M+)

| Analysis for $C_{34}H_{56}N_2O_6$: | | | |
|---|---|---|---|
| Theory: | C, 69.35; | H, 9.59; | N, 4.76. |
| Found: | C, 69.71; | H, 9.85; | N, 4.79. |

Example 35

Synthesis of (2R,4R) 4-amino-1-(4-dodecylbenzyl)pyrrolidine-2,4-dicarboxylic acid

The compound of Example 34 (0.39 g, 0.66 mmol) was stirred in ethyl acetate chilled to 0°C. Anhydrous hydrogen chloride was then bubbled through the solution until saturated.

The reaction mixture warmed to room temperature and then stirred at room temperature for about three hours. The reaction mixture was concentrated to dryness, reconstituted in tetrahydrofuran (15 ml) and 1 N sodium hydroxide (15 ml), and then stirred at room temperature for two days.

The tetrahydrofuran was removed in vacuo, and the aqueous solution was acidified until precipitate forms (about pH 2.0). The solids were removed by filtration and washed with isopropyl alcohol, followed by a wash with diethyl ether to yield 0.12 grams (39%) of the hydrochloride salt of the title product as a white solid. FDMS 433 (M+).

| Analysis for $C_{25}H_{40}N_2O_4 \cdot HCl$: | | | |
|---|---|---|---|
| Theory: | C, 64.07; | H, 8.60; | N, 5.98. |
| Found: | C, 63.09; | H, 8.95; | N, 5.82. |

Example 36

Synthesis of (2R,4R) diethyl-4-(*tert*-butyloxycarbonylamino)-1-[6,7-dimethoxycoumarin-4-yl)methyl] pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.39 g, 3.02 mmol), 4-(bromomethyl)-6,7-dimethoxycoumarin (0.68 g, 2.27 mmol), and tetrabutylammonium iodide (0.56 g, 1.51 mmol) were dissolved in methylene chloride (25 ml). The resulting mixture was then stirred under a nitrogen atmosphere at room temperature for about three days.

The reaction mixture was then partitioned between 0.5 N sodium hydroxide and methylene chloride. The organic fraction was removed. The aqueous fraction was extracted with ether, and the extract was combined with the earlier organics. The combined organic fraction was washed with brine, dried over potassium carbonate, and then concentrated

in vacuo. The desired product was further purified by recrystallizing from ethyl acetate. The solid (coumarin starting material) was discarded. The filtrate was concentrated by vacuum and further purified by thin layer chromatography to yield 0.77 g (95%) of the title product as a light yellow solid. FDMS 548 (M+)

| Analysis for $C_{27}H_{36}N_2O_{10}$: | | | |
|---|---|---|---|
| Theory: | C, 59.11; | H, 6.61; | N, 5.11. |
| Found: | C, 63.05; | H, 7.19; | N, 4.34. |

Example 37

Synthesis of (2R,4R) 4-amino-1-[6,7-dimethoxycoumarin-4-yl)methyl]pyrrolidine-2,4-dicarboxylic acid

The compound of Example 36 (0.68 g, 1.24 mmol) was stirred in ethyl acetate chilled to 0°C. Anhydrous hydrogen chloride was then bubbled through the solution until saturated.

The reaction mixture warmed to room temperature and then stirred at room temperature for about two hours. The reaction mixture was concentrated to dryness, reconstituted in tetrahydrofuran (15 ml) and 1 N sodium hydroxide (15 ml), and then stirred at room temperature for two days.

The reaction mixture was acidified to pH 10 with 1 N hydrochloric acid and then applied to an anion exchange column, eluting with 3 N acetic acid. The desired product was further purified as the ammonium salt by thin layer chromatography. The pH of this ammonium salt was then adjusted to pH 9.0 and applied to an anion exhange column, again eluting with 3 N acetic acid to yield 0.31 grams (64%) of the title product as a light yellow solid.

| Analysis for $C_{18}H_{20}N_2O_8$: | | | |
|---|---|---|---|
| Theory: | C, 55.10; | H, 5.14; | N, 7.14. |
| Found: | C, 57.42; | H, 5.77; | N, 7.32. |

Example 38

Synthesis of (2R,4R) diethyl-4-(*tert*-butyloxycarbonylamino)-1-(3,4-dichlorobenzyl)pyrrolidine-2,4-dicarboxylate

An amount of (2R,4R) diethyl-4-(*tert* butyloxycarbonylamino)pyrrolidine-2,4-dicarboxylate (0.50 g, 1.51 mmol), Hunig's base (N,N-diisopropylethylamine) (0.4 g, 3.05 mmol), 3,4-dichlorobenzyl chloride (0.44 g, 2.23 mmol), and tetrabutylammonium iodide (0.56 g, 1.51 mmol) were dissolved in methylene chloride (25 ml). The resulting mixture was then stirred under a nitrogen atmosphere at room temperature overnight, after which time the reaction mixture was twice briefly heated to reflux and then allowed to recool to room temperature. The reaction mixture was then stirred at room temperature overnight.

The reaction mixture was then partitioned between 0.5 N sodium hydroxide and methylene chloride. The organic fraction was removed. The aqueous fraction was extracted with ether, and the extract was combined with the earlier organics. The combined organic fraction was washed with brine, dried over potassium carbonate, and then concentrated in vacuo. The desired product was further purified by thin layer chromatography to yield 0.65 g (88%) of the title product as a colorless oil which solidified upon standing to a waxy solid. FDMS 488 (M+)

| Analysis for $C_{22}H_{30}Cl_2N_2O_6$: | | | |
|---|---|---|---|
| Theory: | C, 53.99; | H, 6.18; | N, 5.72. |
| Found: | C, 54.21; | H, 6.22; | N, 5.75. |

Example 39

Synthesis of (2R,4R) 4-amino-1-(3,4-dichlorobenzyl)pyrrolidine-2,4-dicarboxylic acid

The compound of Example 38 (0.55 g, 1.12 mmol) was stirred in ethyl acetate chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (15 ml) and 1 N sodium hydroxide (15 ml), and then stirred at room temperature for two days.

The tetrahydrofuran was removed by vacuum, the reaction mixture was acidified to pH 10 with 1 N hydrochloric acid, and then applied to an anion exchange column, eluting with 3 N acetic acid. The desired product was further purified by recrystallization from hot ispropyl alcohol/water to afford 0.19 grams. The filtrate was concentrated to dryness and recrystallization from ethyl acetate/isopropyl alcohol/water yielded an additional 0.08 grams of desired title product. Total yield: 0.27 grams (51%).

| Analysis for $C_{13}H_{14}Cl_2N_2O_4$: | | | |
|---|---|---|---|
| Theory: | C, 46.87; | H, 4.24; | N, 8.41. |
| Found: | C, 46.62; | H, 4.15; | N, 8.23. |

Example 40

Synthesis of (2R,4R) diethyl-4-(*tert*-butyloxycarbonylamino)-1-(2,5-dichlorobenzyl)pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.4 g, 3.05 mmol), 2,5-dichlorobenzyl chloride (0.44 g, 2.23 mmol), and tetrabutylammonium iodide (0.56 g, 1.51 mmol) were dissolved in methylene chloride (25 ml). The resulting mixture was then stirred under a nitrogen atmosphere at room temperature overnight, after which time the reaction mixture was twice briefly heated to reflux and then allowed to recool to room temperature. The reaction mixture was then stirred at room temperature overnight.

The reaction mixture was then partitioned between 0.5 N sodium hydroxide and methylene chloride. The organic fraction was removed. The aqueous fraction was extracted with ether, and the extract was combined with the earlier organics. The combined organic fraction was washed with brine, dried over potassium carbonate, and then concentrated in vacuo. The desired product was further purified by thin layer chromatography to yield 0.79 g (>99%) of the title product as a colorless oil. FDMS 488 (M+)

| Analysis for $C_{22}H_{30}Cl_2N_2O_6$: | | | |
|---|---|---|---|
| Theory: | C, 53.99; | H, 6.18; | N, 5.72. |
| Found: | C, 54.16; | H, 6.31; | N, 5.52. |

Example 41

Synthesis of (2R,4R) 4-amino-1-(2,5-dichlorobenzyl)pyrrolidine-2,4-dicarboxylic acid

The compound of Example 40 (0.65 g, 1.3 mmol) was stirred in ethyl acetate chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (15 ml) and 1 N sodium hydroxide (15 ml), and then stirred at room temperature for three days.

The tetrahydrofuran was removed by vacuum, the reaction mixture was acidified to pH 10 with 1 N hydrochloric acid, and then applied to an anion exchange column, eluting with 3 N acetic acid. The desired product was further purified by recrystallization from hot ispropyl alcohol/water (10 ml total volume) to afford 0.35 grams (81%). FDMS 333 (M+1).

| Analysis for $C_{13}H_{14}Cl_2N_2O_4$: | | | |
|---|---|---|---|
| Theory: | C, 46.87; | H, 4.24; | N, 8.41. |
| Found: | C, 47.08; | H, 4.18; | N, 8.27. |

Example 42

Synthesis of (2R,4R) diethyl-4-(*tert*-butyloxycarbonylamino)-1-(3,4-dibenzyloxybenzyl)pyrrolidine-2,4-dicarboxylate

An amount of (2R,4R) diethyl-4-(*tert*- butyloxycarbonylamino)pyrrolidine-2,4-dicarboxylate (0.50 g, 1.51 mmol), Hunig's base (N,N-diisopropylethylamine) (0.39 g, 3.0 mmol), 3,4-dibenzyloxybenzyl chloride (0.76 g, 2.23 mmol), and tetrabutylammonium iodide (0.56 g, 1.51 mmol) were dissolved in methylene chloride (35 ml). The resulting mixture was

then stirred under a nitrogen atmosphere at room temperature for about three days.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.95 g (>99%) of the title product as a colorless oil.

| Analysis for $C_{36}H_{44}N_2O_8$: | | | |
|---|---|---|---|
| Theory: | C, 63.34; | H, 7.01; | N, 4.43. |
| Found: | C, 63.34; | H, 7.09; | N, 4.35. |

Example 43

Synthesis of (2R,4R) 4-amino-1-(3,4-dibenzyloxybenzyl)pyrrolidine-2,4-dicarboxylic acid

The compound of Example 42 (0.85 g, 1.34 mmol) was stirred in ethyl acetate (40 ml) chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (15 ml) and 1 N sodium hydroxide (15 ml), and then stirred at room temperature overnight.

The reaction mixture was acidified to pH 4 and then stirred overnight at room temperature. The solids were collected by filtration, washed with water, then isopropyl alcohol to yield 0.51 grams (80%). FDMS 477 (M+1).

| Analysis for $C_{27}H_{28}N_2O_6$: | | | |
|---|---|---|---|
| Theory: | C, 68.05; | H, 5.92; | N, 5.88. |
| Found: | C, 69.95; | H, 6.00; | N, 5.67. |

Example 44

Synthesis of (E) (2R,4R) diethyl-4-(*tert*-butyloxycarbonylamino)-1-[4-(2-phenylethenyl)benzyl] pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.39 g, 3.0 mmol), (E) 4-(2-phenylethenyl)benzyl chloride (4-chloromethylstilbene) (0.52 g, 2.23 mmol), and tetrabutylammonium iodide (0.56 g, 1.51 mmol) were dissolved in methylene chloride (35 ml). The resulting mixture was then stirred under a nitrogen atmosphere at room temperature for about three days.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.79 g (>99%) of the title product as a waxy white solid.

| Analysis for $C_{30}H_{38}N_2O_6$: | | | |
|---|---|---|---|
| Theory: | C, 68.94; | H, 7.33; | N, 5.36. |
| Found: | C, 68.66; | H, 7.32; | N, 5.41. |

Example 45

Synthesis of (E) (2R,4R) 4-amino-1-([4-(2-phenylethenyl)benzyl]pyrrolidine-2,4-dicarboxylic acid

The compound of Example 44 (0.70 g, 1.34 mmol) was stirred in ethyl acetate (40 ml) chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (10 ml) and 1 N sodium hydroxide (10 ml), and then stirred at room temperature overnight.

The reaction mixture was adjusted to pH 10 with 1 N hydrochloric acid and then applied to an anion exchnage column, eluting with 50 % acetic acid in water and then 100% acetic acid. Heating of the eluent and the column was necessary to afford displacement. The eluate was concentrated to dryness. The residue was then triturated in hot water. The solids were collected by filtration and washed with water to yield 0.41 grams (84%) of the title product was a white solid. FDMS 367 (M+1).

| Analysis for $C_{21}H_{22}N_2O_4$: | | | |
|---|---|---|---|
| Theory: | C, 68.84; | H, 6.05; | N, 7.65. |
| Found: | C, 68.74; | H, 6.07; | N, 7.41. |

Example 46

Synthesis of (2R,4R) diethyl-4-(*tert*-butyloxycarbonylamino)-1-(2-trifluoromethylbenzyl)pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.39 g, 3.02 mmol), and 2-trifluoromethyl bromide (0.52 g, 2.23 mmol) were dissolved in methylene chloride (25 ml). The resulting mixture was then stirred under a nitrogen atmosphere at room temperature overnight. The reaction mixture was then heated to reflux and then refluxed overnight.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.67 g (91%) of the title product as a colorless oil.

Example 47

Synthesis of (2R,4R) 4-amino-1-(2-trifluoromethylbenzyl)pyrrolidine-2,4-dicarboxylic acid

The compound of Example 46 (0.60 g, 1.23 mmol) was stirred in ethyl acetate (40 ml) chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (10 ml) and 1 N sodium hydroxide (10 ml), and then stirred at room temperature for about three days.

The reaction mixture was adjusted to pH 10 with 1 N hydrochloric acid, the tetrahydrofuran was removed by vacuum, and the reaction mixture was applied to an anion exchnage column, eluting with 50 % acetic acid in water. Heating of the eluent and the column was necessary to afford displacement. The eluate was concentrated to dryness. The residue was then reconstituted in hot water, the pH was adjusted to 9.0, and the solution was applied to an anion exchange column, eluting with 3 N acetic acid to yield 0.31 grams (76%) of the title product was a white solid. FDMS 333 (M+1).

| Analysis for $C_{14}H_{15}F_3N_2O_4$: | | | |
|---|---|---|---|
| Theory: | C, 50.61; | H, 4.55; | N, 8.43. |
| Found: | C, 50.71; | H, 4.52; | N, 8.33. |

Example 48

Synthesis of (2R,4R) diethyl-4-(*tert*-butyloxycarbonylamino)-1-[(2-phenylsulfonylmethyl)benzyl] pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.39 g, 3.02 mmol), and 1-bromomethyl-2-[(phenylsulfonyl)methyl]benzene (0.73 g, 2.23 mmol) were dissolved in methylene chloride (25 ml). The resulting mixture was then stirred under a nitrogen atmosphere at room temperature overnight.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.67 g (91%) of the title product as a colorless

oil.

| Analysis for $C_{29}H_{38}N_2O_8S$: | | | |
|---|---|---|---|
| Theory: | C, 60.61; | H, 6.66; | N, 4.87. |
| Found: | C, 60.79; | H, 6.63; | N, 4.91. |

Example 49

Synthesis of (2R,4R) 4-amino-1-[(2-phenylsulfonylmethyl)benzyl]pyrrolidine-2,4-dicarboxylic acid

The compound of Example 48 (0.75 g, 1.3 mmol) was stirred in ethyl acetate (40 ml) chilled to 0°C. Anhydrous hydrogen chloride was then bubbled through the solution until saturated.

The reaction mixture was allowed to warm to room temperature and then stirred at room temperature for about three hours. The reaction mixture was concentrated to dryness, reconstituted in tetrahydrofuran (10 ml) and 1 N sodium hydroxide (10 ml), and then stirred at room temperature overnight.

The reaction mixture was adjusted to pH 10 with 1 N hydrochloric acid, the tetrahydrofuran was removed by vacuum, and the reaction mixture was applied to an anion exchnage column, eluting with 50 % acetic acid in water to yield 0.54 grams (>99%) of the title product was a white solid. FDMS 419 (M+1).

| Analysis for $C_{20}H_{22}N_2O_6S$: | | | |
|---|---|---|---|
| Theory: | C, 57.41; | H, 5.30; | N, 6.69. |
| Found: | C, 59.06; | H, 5.33; | N, 6.33. |

Example 50

Synthesis of (2R,4R) diethyl-4-(*tert*-butyloxycarbonylamino)-1-(naphthyl-1-ylmethyl)pyrrolidine-2,4-dicarboxylate

An amount of (2R,4R) diethyl-4-(*tert*-butyloxycarbonylamino)pyrrolidine-2,4-dicarboxylate (0.75 g, 2.27 mmol) and Hunig's base (N,N-diisopropylethylamine) (0.60 g, 4.54 mmol) were dissolved in methylene chloride (40 ml) followed by the addition of naphthyl-1-ylmethyl chloride (1-chloromethylnaphthalene) (0.60 g, 3.44 mmol). To this reaction mixture was then added tetrabutylammonium iodide (0.83 g, 2.27 mmol). The resulting mixture was then stirred under a nitrogen atmosphere overnight at room temperature.

The reaction mixture was then partitioned between 0.5 N sodium hydroxide and ethyl acetate. The organic fraction was removed. The aqueous fraction was extracted with ether, and the extract was combined with the earlier organics. The combined organic fraction was extracted with brine, dried over potassium carbonate, and then concentrated in vacuo. The desired product was further purified by thin layer chromatography to yield 0.95 g (89%) of the title product as a colorless oil which solidified upon standing. FDMS 471 (M+1)

| Analysis for $C_{26}H_{34}N_2O_6$: | | | |
|---|---|---|---|
| Theory: | C, 66.36; | H, 7.28; | N, 5.95. |
| Found: | C, 66.15; | H, 7.31; | N, 6.01. |

Example 51

Synthesis of (2R,4R) 4-amino-1-(naphthyl-1-ylmethyl)pyrrolidine-2,4-dicarboxylic acid

The compound of Example 50 (0.85 g, 1.80 mmol) was dissolved in diethyl ether, chilled to 0°C. Anhydrous hydrogen chloride was then bubbled through the solution until saturated.

The reaction mixture was allowed to warm to room temperature and then stirred at room temperature overnight. The reaction mixture was concentrated to dryness, reconstituted in tetrahydrofuran (12.5 ml) and 1 N sodium hydroxide (12.5 ml), and then stirred at room temperature overnight.

The reaction mixture was concentrated to dryness, was reconstituted in water, and the pH was adjusted to 10 with 1 N hydrochloric acid. The reaction mixture was then applied to an anion exchange column, eluting with 50 % acetic acid in water. Recrystallization from water, followed by a wash with isopropyl alcohol, yielded 0.39 grams (69%) of the title product was a white solid. FDMS 270, 315(M+1).

| Analysis for $C_{17}H_{18}N_2O_4$: | | | |
|---|---|---|---|
| Theory: | C, 64.96; | H, 5.77; | N, 8.91. |
| Found: | C, 65.17; | H, 5.58; | N, 8.84. |

Example 52

Synthesis of (2R,4R) diethyl-4-(*tert*-butyloxycarbonylamino)-1-(diphenylmethyl)pyrrolidine-2,4-dicarboxylate

An amount of (2R,4R) diethyl-4-(*tert*-butyloxycarbonylamino)pyrrolidine-2,4-dicarboxylate (0.50 g, 1.5 mmol) and Hunig's base (N,N-diisopropylethylamine) (0.39 g, 3.0 mmol) were dissolved in methylene chloride (35 ml) followed by the addition of bromodiphenylmethane (0.56 g, 2.3 mmol). The resulting mixture was then stirred under a nitrogen atmosphere overnight at room temperature.

The reaction mixture was then partitioned between 1.0 N sodium hydroxide and ethyl acetate. The organic fraction was removed. The aqueous fraction was extracted with ether, and the extract was combined with the earlier organics. The combined organic fraction was extracted with brine, dried over potassium carbonate, and then concentrated in vacuo. The desired product was further purified by thin layer chromatography to yield 0.48 grams (64%) of the title product as a colorless oil. FDMS 496 (M+1)

Example 53

Synthesis of (2R,4R) 4-amino-1-(diphenylmethyl)pyrrolidine-2,4-dicarboxylic acid

The compound of Example 52 (0.40 g, 0.81 mmol) was dissolved in 1 N hydrochloric acid, with ethanol present to enhance solubility. The reaction mixture was stirred at room temperature for about three days.

The reaction mixture was then concentrated to dryness, reconstituted in tetrahydrofuran (12.5 ml) and 1 N sodium hydroxide (12.5 ml), and then stirred at room temperature overnight.

The reaction mixture was again concentrated to dryness, was reconstituted in water, and the pH was adjusted to 10 with 1 N hydrochloric acid. The resulting mixture was then applied to an anion exchange column, eluting with 50 % acetic acid in water, to yield 0.24 grams (87%) of the title product was a white solid. FDMS 341 (M+1).

| Analysis for $C_{19}H_{20}N_2O_4 \cdot H_20$: | | | |
|---|---|---|---|
| Theory: | C, 65.51; | H, 5.63; | N, 7.82. |
| Found: | C, 65.62; | H, 5.71; | N, 7.75. |

Example 54

Synthesis of (2R,4R) diethyl-4-(*tert*-butyloxycarbonylamino)-1-(4-chlorobenzyl)pyrrolidine-2,4-dicarboxylate

An amount of (2R,4R) diethyl-4-(*tert*- butyloxycarbonylamino)pyrrolidine-2,4-dicarboxylate (0.50 g, 1.51 mmol), Hunig's base (N,N-diisopropylethylamine) (0.39 g, 3.0 mmol), 4-chlorobenzyl chloride (0.37 g, 2.27 mmol), and tetrabutylammonium iodide (0.55 g, 1.5 mmol) were dissolved in methylene chloride (35 ml). The resulting mixture was then stirred under a nitrogen atmosphere at room temperature overnight.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.67 g (99%) of the title product as a colorless oil.

| Analysis for $C_{21}H_{31}ClN_2O_6$: | | | |
|---|---|---|---|
| Theory: | C, 58.08; | H, 6.87; | N, 6.16. |
| Found: | C, 57.78; | H, 6.74; | N, 6.08. |

Example 55

Synthesis of (2R,4R) 4-amino-1-(4-chlorobenzyl)pyrrolidine-2,4-dicarboxylic acid

The compound of Example 54 (0.58 g, 1.3 mmol) was stirred in 1 N hydrochloric acid at room temperature overnight to hydrolyze the t-BOC group. The reaction mixture was then concentrated to dryness.

The reaction mixture was concentrated to dryness, reconstituted in tetrahydrofuran (15 ml) and 1 N sodium hydroxide (15 ml), and then stirred at room temperature overnight to saponify the esters. The reaction mixture was again concentrated to dryness.

The residue was reconstituted in water and the mixture was acidified to pH 10 and then applied to an anion exchange column, eluting with 50% acetic acid in water to yield 0.31 grams (80%). FDMS 254, 299(M+).

| Analysis for $C_{13}H_{15}ClN_2O_4 \cdot H_2O$: | | | |
|---|---|---|---|
| Theory: | C, 49.30; | H, 4.77; | N, 8.84. |
| Found: | C, 49.55; | H, 5.18; | N, 8.33. |

Example 56

Synthesis of (2R,4R) diethyl-4-(*tert*-butyloxycarbonylamino)-1-(3-chlorobenzyl)pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol), Hunig's base (N,N-diisopropylethylamine) (0.39 g, 3.0 mmol), 4-chlorobenzyl bromide (0.47 g, 2.27 mmol), and tetrabutylammonium iodide (0.55 g, 1.5 mmol) were dissolved in methylene chloride (35 ml). The resulting mixture was then stirred under a nitrogen atmosphere at room temperature for about three days.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.70 g (>99%) of the title product as a colorless oil. FDMS 454 (M+)

| Analysis for $C_{21}H_{31}ClN_2O_6$: | | | |
|---|---|---|---|
| Theory: | C, 58.08; | H, 6.87; | N, 6.16. |
| Found: | C, 57.95; | H, 6.76; | N, 5.93. |

Example 57

Synthesis of (2R,4R) 4-amino-1-(3-chlorobenzyl)pyrrolidine-2,4-dicarboxylic acid

The compound of Example 56 (0.60 g, 1.3 mmol) was stirred in 1 N hydrochloric acid (with ethanol added to enhance solubility) at room temperature for about three days to hydrolyze the *t*-BOC group.

The reaction mixture was concentrated to dryness, reconstituted in tetrahydrofuran (15 ml) and 1 N sodium hydroxide (15 ml), and then stirred at room temperature overnight to saponify the esters. The reaction mixture was again concentrated to dryness.

The residue was reconstituted in water and the mixture was acidified to pH 10 and then applied to an anion exchange column, eluting with 50% acetic acid in water to yield 0.40 grams (>99%) of the title product. FDMS 254, 299(M+).

| Analysis for $C_{13}H_{15}ClN_2O_4 \cdot H_2O$: | | | |
|---|---|---|---|
| Theory: | C, 49.30; | H, 4.77; | N, 8.84. |
| Found: | C, 48.87; | H, 5.35; | N, 8.36. |

Example 58

Synthesis of (2R,4R) diethyl-4-(*tert*-butyloxycarbonylamino)-1-(2-chlorobenzyl)pyrrolidine-2,4-dicarboxylate

An amount of (2R,4R) diethyl-4-(*tert*- butyloxycarbonylamino)pyrrolidine-2,4-dicarboxylate (0.50 g, 1.51 mmol), Hunig's base (N,N-diisopropylethylamine) (0.39 g, 3.0 mmol), 2-chlorobenzyl chloride (0.37 g, 2.27 mmol), and tetrabutylammonium iodide (0.55 g, 1.5 mmol) were dissolved in methylene chloride (35 ml). The resulting mixture was then stirred under a nitrogen atmosphere at room temperature for about three days. Additional 2-chlorobenzyl chloride and Hunig's base (1.5 mmol of each) were then added and the reaction mixture was stirred overnight at room temperature.

The reaction mixture was then partitioned between 0.5 N sodium hydroxide and methylene chloride. The organic fraction was removed. The aqueous fraction was extracted with ether, and the extract was combined with the earlier organics. The combined organic fraction was dried over potassium carbonate, and then concentrated in vacuo. The desired product was further purified by thin layer chromatography to yield 0.60 g (88%) of the title product as a colorless oil which solidified to a waxy white solid upon standing. mp 48-49°C. FDMS 418, 454(M+)

| Analysis for $C_{21}H_{31}ClN_2O_6$: | | | |
|---|---|---|---|
| Theory: | C, 58.08; | H, 6.87; | N, 6.16. |
| Found: | C, 58.29; | H, 7.06; | N, 6.37. |

Example 59

Synthesis of (2R,4R) 4-amino-1-(2-chlorobenzyl)pyrrolidine-2,4-dicarboxylic acid

The compound of Example 58 (0.50 g, 1.1 mmol) was stirred in 1 N hydrochloric acid (with ethanol added to enhance solubility) at room temperature for about three days to hydrolyze the *t*-BOC group.

The reaction mixture was concentrated to dryness, reconstituted in tetrahydrofuran (15 ml) and 1 N sodium hydroxide (15 ml), and then stirred at room temperature overnight to saponify the esters. The reaction mixture was again concentrated to dryness.

The residue was reconstituted in water and the mixture was acidified to pH 10 and then applied to an anion exchange column, eluting with 50% acetic acid in water to yield 0.32 grams (98%) of the title product. FDMS 299 (M+).

| Analysis for $C_{13}H_{15}ClN_2O_4 \cdot H_2O$: | | | |
|---|---|---|---|
| Theory: | C, 49.30; | H, 4.77; | N, 8.84. |
| Found: | C, 50.38; | H, 5.04; | N, 8.44. |

Example 60

Synthesis of (2R,4R) diethyl-4-(*tert*-butyloxycarbonylamino)-1-(3,3-diphenylpropyl)pyrrolidine-2,4-dicarboxylate

An amount of (2R,4R) diethyl-4-(*tert*-butyloxycarbonylamino)pyrrolidine-2,4-dicarboxylate (0.50 g, 1.51 mmol) was dissolved in acetonitrile (35 ml) as Hunig's base (N,N-diisopropylethylamine) (0.40 g, 3.0 mmol) and 3,3-diphenyl-1-iodo-propane were added consecutively. The resulting mixture was then stirred at room temperature overnight. The mixture was then heated to 60°C and then stirred an additional overnight.

The reaction mixture was partitioned between 1 N sodium hydroxide and diethyl ether. The aqueous fraction was extracted with diethyl ether. The organic fractions were combined, dried over potassium carbonate, and the solvents were removed in vacuo.

The desired product was further purified by thin layer chromatography to yield 0.75 grams (95%) of the title product as a colorless oil. FDMS 524 (M+).

| Analysis for $C_{30}H_{40}N_2O_6$: | | | |
|---|---|---|---|
| Theory: | C, 68.68; | H, 7.68; | N, 5.34. |
| Found: | C, 68.75; | H, 7.47; | N, 5.26. |

Example 61

Synthesis of (2R,4R) 4-amino-1-(2-chlorobenzyl)pyrrolidine-2,4-dicarboxylic acid

The compound of Example 60 (0.65 g, 1.24 mmol) was dissolved in diethyl ether, chilled to 0°C. Anhydrous hydrogen chloride was then bubbled through the solution until saturated.

The reaction mixture was allowed to warm to room temperature and then stirred at room temperature overnight.

The reaction mixture was concentrated to dryness, reconstituted in tetrahydrofuran (20 ml) and 1 N sodium hydroxide (20 ml), and then stirred at room temperature overnight.

The reaction mixture was concentrated to dryness, was reconstituted in water, and the pH was adjusted to 10 with 1 N hydrochloric acid. The reaction mixture was then applied to an anion exchange column, eluting with 50 % acetic acid in water. The leuate was concentrated to dryness, the residual acetic acid being removed by azeotope with water, then isopropyl alcohol. The residue was triturated with water and isopropyl alcohol, and then diethyl ether to yield 0.33 grams (72%) of the title product was a white solid. FDMS 369 (M+1).

| Analysis for $C_{17}H_{18}N_2O_4$: | | | |
|---|---|---|---|
| Theory: | C, 68.46; | H, 6.57; | N, 7.60. |
| Found: | C, 65.24; | H, 6.59; | N, 7.20. |

Example 62

Synthesis of (2R,4R) diethyl-4-(*tert*-butyloxycarbonylamino)-1-(3,3-diphenylpropanoyl)pyrrolidine-2,4-dicarboxylate

In pyridine (15 ml) were stirred 3,3-diphenylpropionic acid (0.62 g, 2.7 mmol) and 1-cyclohexyl-3-(2-morpholinoethyl) carbodiimide methyl-*p*-toluenesulfonate (1.15 g, 2.7 mmol). To this mixture was added (2R,4R) diethyl-4-(*tert*-butyloxycarbonylamino)pyrrolidine-2,4-dicarboxylate (0.60 g, 1.8 mmol) in several portions. The reaction mixture was then stirred for about four days.

The reaction mixture was then acidified with 6 N hydrochloric acid and partitioned with diethyl ether. The aqueous fraction was extracted with diethyl ether. The organic fractions were combined and washed with 1 N hydrochloric acid. The organic fraction was extracted with 1 N sodium hydroxide, then brine, and then dried voer potassium carbonate. The solvents were removed in vacuo.

The desired product was further purified by thin layer chromatography to yield 0.86 grams (89%) of the title product as a white foam.

| Analysis for $C_{30}H_{38}N_2O_7$: | | | |
|---|---|---|---|
| Theory: | C, 66.90; | H, 7.11; | N, 5.20. |
| Found: | C, 66.60; | H, 7.11; | N, 5.15. |

Example 63

Synthesis of (2R,4R) 4-amino-1-(3,3-diphenylpropanoyl)pyrrolidine-2,4-dicarboxylic acid

The compound of Example 61 (0.73 g, 1.36 mmol) was dissolved in diethyl ether, chilled to 0°C. Anhydrous hydrogen chloride was then bubbled through the solution until saturated.

The reaction mixture was allowed to warm to room temperature and then stirred at room temperature for one hour. The reaction mixture was concentrated to dryness, reconstituted in tetrahydrofuran (20 ml) and 1 N sodium hydroxide (20 ml), and then stirred at room temperature overnight.

The reaction mixture was again concentrated to dryness, was reconstituted in water, and the pH was adjusted to 10 with 1 N hydrochloric acid. The reaction mixture was then applied to an anion exchange column, eluting with 3 N acetic acid. The eluate was concentrated to dryness, the residual acetic acid being removed by azeotope with water, then isopropyl alcohol. The residue was triturated with water to yield 0.44 grams (85%) of the title product was a white solid. FDMS 383 (M+1).

| Analysis for $C_{21}H_{22}N_2O_5$: | | | |
|---|---|---|---|
| Theory: | C, 65.96; | H, 5.80; | N, 7.33. |
| Found: | C, 65.72; | H, 5.70; | N, 7.14. |

Example 64

Synthesis of (2R,4R) diethyl-4-(*tert*-butyloxycarbonylamino)-1-(diphenylacetyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.60 g, 1.8 mmol) was stirred at room temperature as Hunig's base (N,N-diisopropylethylamine) (0.50 g, 3.8 mmol) and diphenylacetyl chloride (0.38 g, 1.65 mmol) were added consecutively. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over potassium carbonate. The solvents were removed in vacuo.

The desired product was further purified by thin layer chromatography to yield 0.78 grams (99%) of the title product as a yellow foam. FDMS 524 (M+).

| Analysis for $C_{29}H_{36}N_2O_7$: | | | |
|---|---|---|---|
| Theory: | C, 66.40; | H, 6.92; | N, 5.34. |
| Found: | C, 66.02; | H, 7.20; | N, 4.72. |

Example 65

Synthesis of (2R,4R) 4-amino-1-(diphenylacetyl)pyrrolidine-2,4-dicarboxylic acid

The compound of Example 64 (0.72 g, 1.37 mmol) was dissolved a 1:1 mixture of 1 N sodium hydroxide (15 ml) and tetrahydrofuran (15 ml). This oslution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water, to yield 0.42 grams (83%) of the title product as a white solid. FDMS 368 (M+).

| Analysis for $C_{20}H_{20}N_2O_5$: | | | |
|---|---|---|---|
| Theory: | C, 65.21; | H, 5.47; | N, 7.60. |
| Found: | C, 64.58; | H, 5.43; | N, 7.15. |

Example 66

Synthesis of (2R,4R) diethyl-4-(*tert*-butyloxycarbonylamino)-1-(3-phenylpropanoyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.60 g, 1.8 mmol) was stirred at room temperature as Hunig's base (N,N-diisopropylethylamine) (0.50 g, 3.8 mmol) and 3-phenylpropanoyl chloride (hydrocinnamoyl chloride) (0.27 g, 1.65 mmol) were added consecutively. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over magnesium sulfate. The solvents were removed in vacuo.

The desired product was further purified by thin layer chromatography to yield 0.64 grams (92%) of the title product as a colorless oil. FDMS 462 (M+).

| Analysis for $C_{24}H_{34}N_2O_7$: | | | |
|---|---|---|---|
| Theory: | C, 62.32; | H, 7.41; | N, 6.06. |
| Found: | C, 64.14; | H, 7.35; | N, 5.61. |

Example 67

Synthesis of (2R,4R) 4-amino-1-(3-phenylpropanoyl)pyrrolidine-2,4-dicarboxylic acid

The compound of Example 66 (0.55 g, 1.19 mmol) was dissolved a 1:1 mixture of 1 N sodium hydroxide (15 ml)

and tetrahydrofuran (15 ml). This solution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water, to yield 0.34 grams (93%) of the title product as a white solid. FDMS 306 (M+).

| Analysis for $C_{15}H_{18}N_2O_5$: | | | |
|---|---|---|---|
| Theory: | C, 58.82; | H, 5.92; | N, 9.15. |
| Found: | C, 57.29; | H, 5.86; | N, 9.62. |

Example 68

Synthesis of (2R,4R) diethyl-4-(*tert*-butyloxycarbonylamino)-1-(cyclohexylcarbonyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.50 g, 1.5 mmol) was stirred at room temperature as Hunig's base (N,N-diisopropylethylamine) (0.50 g, 3.8 mmol) and cyclohexanecarbonyl chloride (0.24 g, 1.65 mmol) were added consecutively. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over magnesium sulfate. The solvents were removed in vacuo.

The desired product was further purified by thin layer chromatography to yield 0.67 grams (>99%) of the title product as a colorless oil. FDMS 440, 441.

| Analysis for $C_{22}H_{36}N_2O_7$: | | | |
|---|---|---|---|
| Theory: | C, 59.98; | H, 8.24; | N, 6.36. |
| Found: | C, 61.08; | H, 8.38; | N, 6.31. |

Example 69

Synthesis of (2R,4R) 4-amino-1-(cyclohexylcarbonyl)pyrrolidine-2,4-dicarboxylic acid

The compound of Example 68 (0.60 g, 1.36 mmol) was dissolved a 1:1 mixture of 1 N sodium hydroxide (15 ml) and tetrahydrofuran (15 ml). This solution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water, to yield 0.35 grams (91%) of the title product as a white solid. FDMS 285 (M+).

| Analysis for $C_{13}H_{20}N_2O_5$: | | | |
|---|---|---|---|
| Theory: | C, 54.92; | H, 7.09; | N, 9.85. |
| Found: | C, 54.78; | H, 7.12; | N, 9.98. |

Example 70

Synthesis of (2R,4R) diethyl-4-(*tert*-butyloxycarbonylamino)-1-(phenylacetyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.50 g, 1.5 mmol) was stirred at room temperature as Hunig's base (N,N-diisopropylethylamine) (0.50 g, 3.8 mmol) and phenylacetyl chloride (0.26 g, 1.65 mmol) were added consecutively. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over magnesium sulfate. The solvents were removed in vacuo.

The desired product was further purified by thin layer chromatography to yield 0.58 grams (86%) of the title product as a colorless oil. FDMS 448, 449.

| Analysis for $C_{23}H_{32}N_2O_7$: | | | |
|---|---|---|---|
| Theory: | C, 61.59; | H, 7.19; | N, 6.25. |
| Found: | C, 61.36; | H, 6.93; | N, 6.48. |

Example 71

Synthesis of (2R,4R) 4-amino-1-(phenylacetyl)pyrrolidine-2,4-dicarboxylic acid

The compound of Example 70 (0.53 g, 1.18 mmol) was dissolved a 1:1 mixture of 1 N sodium hydroxide (15 ml) and tetrahydrofuran (15 ml). This solution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water, to yield 0.33 grams (96%) of the title product as a white solid. FDMS 293 (M+1).

| Analysis for $C_{14}H_{16}N_2O_5$: | | | |
|---|---|---|---|
| Theory: | C, 57.53; | H, 5.52; | N, 9.58. |
| Found: | C, 53.11; | H, 5.87; | N, 9.75. |

Example 72

Synthesis of (2R,4R) diethyl-4-(*tert*-butyloxycarbonylamino)-1-(3-phenylprop-2-enoyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.50 g, 1.5 mmol) was stirred at room temperature as Hunig's base (N,N-diisopropylethylamine) (0.50 g, 3.8 mmol) and 3-phenylprop-2-enoyl chloride (cinnamoyl chloride) (0.27 g, 1.65 mmol) were added consecutively, the 3-phenylprop-2-enoyl chloride being added dropwise. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over magnesium sulfate. The solvents were removed in vacuo.

The desired product was further purified by thin layer chromatography to yield 0.61 grams (88%) of the title product as a white solid. FDMS 460 (M+).

| Analysis for $C_{24}H_{32}N_2O_7$: | | | |
|---|---|---|---|
| Theory: | C, 62.59; | H, 7.00; | N, 6.08. |
| Found: | C, 62.77; | H, 6.84; | N, 6.01. |

Example 73

Synthesis of (2R,4R) 4-amino-1-(3-phenylprop-2-enoyl)pyrrolidine-2,4-dicarboxylic acid

The compound of Example 72 (0.55 g, 1.19 mmol) was dissolved a 1:1 mixture of 1 N sodium hydroxide (15 ml) and tetrahydrofuran (15 ml). This solution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water, to yield 0.26 grams (72%) of the title product as a white solid. FDMS 259, 304.

| Analysis for $C_{15}H_{16}N_2O_5$: | | | |
|---|---|---|---|
| Theory: | C, 59.21; | H, 5.30; | N, 9.21. |
| Found: | C, 56.54; | H, 5.70; | N, 9.65. |

Example 74

Synthesis of (2R,4R) diethyl-4-(*tert*-butyloxycarbonylamino)-1-(benzoyl)pyrrolidine-2,4-dicarboxylate

In methylene chloride (30 ml dried over molecular sieves) (2R,4R) diethyl-4-(*tert*-butyloxycarbonylamino)pyrrolidine-2,4-dicarboxylate (0.50 g, 1.5 mmol) was stirred at room temperature as Hunig's base (N,N-diisopropylethylamine) (0.50 g, 3.8 mmol) and benzoyl chloride (0.27 g, 1.65 mmol) were added consecutively, the benzoyl chloride being added dropwise. The resulting mixture was then stirred at room temperature for about three days.

The reaction mixture was partitioned between 1 N hydrochloric acid and methylene chloride. The organic fraction was removed and the aqueous fraction was extracted with diethyl ether. The extract was combined with the prior organic fraction and this was dried over magnesium sulfate. The solvents were removed in vacuo.

The desired product was further purified by thin layer chromatography to yield 0.67 grams (>99%) of the title product as a colorless oil. FDMS 434 (M+).

| Analysis for $C_{22}H_{30}N_2O_7$: | | | |
|---|---|---|---|
| Theory: | C, 60.82; | H, 6.96; | N, 6.45. |
| Found: | C, 60.83; | H, 6.75; | N, 6.31. |

Example 75

Synthesis of (2R,4R) 4-amino-1-(benzoyl)pyrrolidine-2,4-dicarboxylic acid

The compound of Example 74 (0.55 g, 1.27 mmol) was dissolved a 1:1 mixture of 1 N sodium hydroxide (15 ml) and tetrahydrofuran (15 ml). This solution was stirred overnight at room temperature to saponify the esters.

The reaction mixture was extracted with diethyl ether. The aqueous fraction was acidified with sufficient 6 N hydrochloric acid to reduce the pH to 1. The aqueous fraction was again extracted with diethyl ether. The organic fractions were combined and dried over magnesium sulfate. The solvents were removed in vacuo.

The residue was reconstituted in diethyl ether and cooled to 0°C. Anhydrous hydrogen chloride gas was bubbled into the solution until the solution was saturated. The reaction mixture was then allowed to warm to room temperature stirred for about thirty minutes.

The reaction mixture was concentrated to dryness, reconstituted in water, and applied to a cation exchange column, eluting with 5% pyridine in water

| Analysis for $C_{13}H_{14}N_2O_5$: | | | |
|---|---|---|---|
| Theory: | C, 56.11; | H, 5.07; | N, 10.07. |
| Found: | C, 52.36; | H, 5.37; | N, 10.52. |

The biological activity of the compounds of the present invention was evaluated employing an initial screening assay which rapidly and accurately measured the binding of the tested compound to known glutamate receptor sites. Assays useful for evaluating glutamate receptor antagonists are well known in the art. See. e.g., D. Schoepp, et al., Neuroscience Letters, 145:100-104 (1992); and R. Wright, et al., Journal of Neurochemistry, 63:938-945 (1994).

The affinity of the compounds for metabotropic glutamate receptors was demonstrated by the selective displacement of (1*S*,3*R*)-1-aminocyclopentane-1,3-dicarboxylic acid-sensitive [$^3$H]glutamate binding to rat brain cell membranes. The binding of [$^3$H] glutamate ([$^3$H]Glu) was conducted with crude membranes of rat forebrain as described by Schoepp and True. Schoepp and True, Neuroscience Letters, 145:100-104 (1992); Wright, et al., Journal of Neurochemistry, 63:938-945 (1994).

## Binding to a Cloned Subtype of Metabotropic Receptor

In an alternative assay suspension cells stably expressing a cloned human mGluR receptor are harvested by centrifugation at 2200 x g for 15 minutes at 4 °C. Membranes for the binding assays are prepared by vortexing the cell pellet in 50 mM Tris.HCl, pH 7.4 (0.5 x $10^9$ cells/30 ml). The tissue suspension is then centrifuged at 39,800 x g for 10 minutes at 4 °C. This procedure is repeated for a total of three washes, with a 10 minute incubation at 37°C between the second and third washes. The final pellet is homogenized in 67 mM Tris·HCl, pH 7.4, at 12.5 x $10^6$ cells/ml using a Tissumizer® (Tekmar, Cincinati, Ohio) at setting 65 for 15 seconds.

Binding assays are performed in triplicate in 0.8 ml total volume. Volumes of 200 µl of membrane suspension (0.07-0.10 mg of protein) and 200 µl of drug dilution in water are added to 400 µl of 67 mM of Tris·HCl, pH 7.4, containing [$^3$H]glutamate (35 nM final concentration, 23.7 Ci/mole), calcium chloride (3 mM), pargyline (10 µM, and Lascorbic acid (5.7 nM). The reaction mixtures are incubated at 37°C for 15 minutes and then rapidly filtered, using a Brandel™ cell harvester (Model MB-48R; Brandel, Gaithersburg, Maryland) over Whatman GF/B filters that had been presoaked in 0.5% polyethyleneimine and precooled with ice-cold 50 mM Tris·HCl, pH 7.4. The filters are then washed rapidly times with ice-cold (4 x 1 ml each).

The amount of [$^3$H]glutamate trapped on the filters is determined by liquid scintillation counting. For the competition experiments, six concentrations of displacing drugs are used, ranging from $10^{-5}$ to $10^{-10}$ M. The $IC_{50}$ values are determined by nonlinear regression analysis (Systat™; Systat Inc., Evanston, Illinois) which may be converted to $K_i$ values using the Cheng-Prusoff equation. Y. Cheng and W.H. Prusoff, Biochemical Pharmacology, 22:3099-3108 (1973).

This type of assay, employing different subtypes of cloned metabotrapic receptors, may be used to determine which compounds have selective affinity in that they bind to one subtype of receptor with a greater affinity than another subtype.

In addition to the binding assays described supra, many of the compounds were also tested for their ability to inhibit the phosphoinositide hydrolysis mediated by the known agonist (1S,3R) ACPD.

## Inhibition of Phosphatidylinositide Hydrolysis

Corss-chopped (0.3 mm) slices of hippocampus from neonatal (10 day old) or adult (55-65 day old) Sprague-Dawley rats wre used to measure [$^3$H]phosphoinositide hydrolysis using standard techniques. See, D.D. Schoepp and B.G. Johnson, Journal of Neurochemistry, 53:1865 (1989). Slices were incubated with [$^3$H]myo-inositol (10 µCi/ml tissue) in a shaking water bath at 37°C. Tissue was washed twice with fresh buffer then further incubated with 10 mM nonlabeled myo-inositol and 10 mM lithium chloride for 20 minutes.

[$^3$H]Phosphoinositide-prelabeled slices were then incubated in the absence (basal) or presence of varioius concentrations of 1S,3R-ACPD or 1R,3S-ACPD for 60 minutes. Incubation buffer also contained 100 µM 1-amino-5-phosphonovalerate and 5 µM staurosporine to prevent NMDA receptor- or protein kinase C-mediated inhibitions of receptor coupling.

[$^3$H]Phosphoinositide hydrolysis was indexed by the amount of [$^3$H]inositol monophosphate formed. [$^3$H]Inositol monophosphate was isolated by anion exchange column chromatography and quantified by liquid scintillation spectrometry.

Many of the compounds employed in the methods of the present invention were tested for their ability to inhibit the phosphoinositide hydrolysis mediated by the known agonist (1S,3R) ACPD. Many of the compounds of Formula I were able to antagonize this response.

In another assay method many of the compounds of Formula I were demonstrated to be effective in the inhibiting ACPD-induced limbic seizures. See. e.g., J. Tizzano, et al., Neuroscience Letters, 162:12-16 (1993). A variety of physiological functions have been shown to be subject to influence by excessive or inappropriate stimulation of excitatory amino acid transmission. The Formula I compounds of the present invention are believed to have the ability to treat a variety of neurological disorders in mammals associated with this condition, including acute neurological disorders such as cerebral deficits subsequent to cardiac bypass surgery and grafting, cerebral ischemia (e.g. stroke and cardiac arrest), spinal cord trauma, head trauma, perinatal hypoxia, and hypoglycemic neuronal damage. The Formula I compounds

are believed to have the ability to treat a variety of chronic neurological disorders, such as Alzheimer's disease, Huntington's Chorea, amyotrophic lateral sclerosis, AIDS-induced dementia, ocular damage and retinopathy, cognitive disorders, and idopathic and drug-induced Parkinson's. The present invention also provides methods for treating these disorders which comprises administering to a patient in need thereof an effective amount of a compound of Formula I.

The Formula I compounds of the present invention are also believed to have the ability to treat a variety of other neurological disorders in mammals that are associated with glutamate dysfunction, including muscular spasms, convulsions, migraine headaches, urinary incontinence, psychosis, drug tolerance, withdrawal, and cessation (i.e. opiates, benzodiazepines, nicotine, cocaine, or ethanol), smoking cessation, anxiety and related disorders (e.g. panic attack), emesis, brain edema, chronic pain, sleep disorders, Tourette's syndrome, attention deficit disorder, and tardive dyskinesia. Therefore, the present invention also provides methods for treating these disorders which comprise administering to a patient in need thereof an effective amount of the compound of Formula I.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also includes pharmaceutical compositions which contain, as the active ingredient, the compounds of Formula I associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 0.05 to about 100 mg, more usually about 1.0 to about 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range. For examples, dosages per day normally fall within the range of about 0.01 to about 30 mg/kg of body weight. In the treatment of adult humans, the range of about 0.1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 120 mg.

Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Active Ingredient | 50.0 mg |
| Xantan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 425 mg quantities.

Formulation Example 9

An intravenous formulation may be prepared as follows:

| Ingredient | Quantity |
|---|---|
| Active Ingredient | 250.0 mg |
| Isotonic saline | 1000 ml |

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
|---|---|
| Active Ingredient | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See. e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by reference.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug

more lipid soluble and amenable to transportation across the blood-brain barrier. Alternatively, the delivery of hydrophilic drugs may be enhanced by intra-arterial infusion of hypertonic solutions which can transiently open the blood-brain barrier.

## Claims

**1.** A compound of the formula

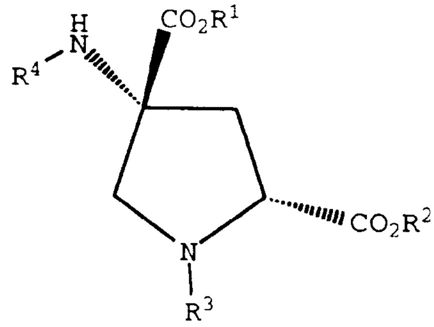

wherein:

$R^1$ and $R^2$ are each individually H or a carboxy protecting group;

$R^4$ is hydrogen or an amino-protecting group; and

$R^3$ is $C_1$-$C_{16}$ alkyl, $C_3$-$C_8$ cycloalkyl, $C_3$-$C_8$ cycloalkenyl, aryl, $C_2$-$C_6$ alkanoyl, $C_2$-$C_6$ alkenoyl, phenyl($C_2$-$C_6$ alkanoyl)-, diphenyl($C_2$-$C_6$ alkanoyl)-, phenyl($C_2$-$C_6$ alkenoyl)-, diphenyl($C_2$-$C_6$ alkenoyl)-, $C_2$-$C_{10}$ alkenyl, $C_2$-$C_{10}$ alkynyl, sulfonyl, aryl($C_1$-$C_6$ alkylidenyl)-, diaryl($C_1$-$C_6$ alkylidenyl)-, (4-phenyl)phenyl($C_1$-$C_6$ alkylidenyl)-, (4-phenyl) phenyl($C_2$-$C_6$ alkanoyl)-, heterocyclic, heterocyclic($C_1$-$C_6$ alkylidenyl)-, or a substituted derivative thereof;

or a salt or solvate thereof.

**2.** A compound as claimed in Claim 1 wherein $R^1$ and $R^2$ are each hydrogen, or a salt or solvate thereof.

**3** A compound as claimed in Claim 2 wherein $R^3$ is $C_1$-$C_6$ alkyl, $C_4$-$C_7$ cycloalkyl, $C_4$-$C_8$ cycloalkenyl, phenyl, naphthyl, $C_2$-$C_6$ alkanoyl, $C_2$-$C_6$ alkenoyl, phenyl($C_2$-$C_6$ alkanoyl)-, diphenyl($C_2$-$C_6$ alkanoyl)-, (4-phenyl)phenyl-, (2-phenyl)phenyl-, (4-phenyl)phenyl($C_1$-$C_6$ alkylidenyl)-, (2-phenyl)phenyl($C_1$-$C_6$ alkylidenyl)-, phenyl($C_2$-$C_6$ alkenoyl)-, diphenyl($C_2$-$C_6$ alkenoyl)-, $C_3$-$C_8$ alkenyl, $C_3$-$C_8$ alkynyl, methylsulfonyl, phenyl($C_1$-$C_6$ alkylidenyl)-, diphenyl($C_1$-$C_6$ alkylidenyl)-, indole, benzo[b]thienyl, indole($C_1$-$C_6$ alkylidenyl)-, benzo[b]thienyl($C_1$-$C_6$ alkylidenyl)-, benzofuranyl ($C_1$-$C_6$ alkylidenyl)-, or a substituted derivative thereof, or a salt or solvate thereof.

**4.** A compound as claimed in Claim 1 wherein at least one of $R^1$ and $R^2$ is not hydrogen, or a salt or solvate thereof.

**5.** A compound as claimed in Claim 4 wherein $R^3$ is $C_1$-$C_6$ alkyl, $C_4$-$C_7$ cycloalkyl, $C_4$-$C_8$ cycloalkenyl, phenyl, naphthyl, $C_2$-$C_6$ alkanoyl, $C_2$-$C_6$ alkenoyl, phenyl($C_2$-$C_6$ alkanoyl)-, diphenyl($C_2$-$C_6$ alkanoyl)-, (4-phenyl)phenyl-, (2-phenyl)phenyl-, (4-phenyl)phenyl($C_1$-$C_6$ alkylidenyl)-, (2-phenyl)phenyl($C_1$-$C_6$ alkylidenyl)-, phenyl($C_2$-$C_6$ alkenoyl)-, diphenyl($C_2$-$C_6$ alkenoyl)-, $C_3$-$C_8$ alkenyl, $C_3$-$C_8$ alkynyl, methylsulfonyl, phenyl($C_1$-$C_6$ alkylidenyl)-, diphenyl($C_1$-$C_6$ alkylidenyl)-, indole, benzo[b]thienyl, indole($C_1$-$C_6$ alkylidenyl)-, benzo[b]thienyl($C_1$-$C_6$ alkylidenyl)-,benzofuranyl($C_1$-$C_6$ alkylidenyl)-, or a substituted derivative thereof, or a salt or solvate thereof.

**6.** A pharmaceutical formulation comprising as an active ingredient a compound as claimed in any one of Claims 1 to 5, or a pharmaceutically acceptable salt or solvate thereof, in combination with one or more pharmaceutically acceptable carriers, diluents, or excipients therefor.

**7.** The use of a compound as claimed in any one of Claims 1 to 5 for use in treating or preventing a physiological condition associated with an inappropriate stimulation of a glutamate receptor in a mammal.

European Patent Office

# EUROPEAN SEARCH REPORT

Application Number
EP 95 30 5800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | CHEMICAL ABSTRACTS, vol. 120, no. 17, 25 April 1994 Columbus, Ohio, US; abstract no. 209158y, * abstract * & BIOCHEM. PHARMACOL., vol. 47, no. 2, 1994 pages 267-274, R. GRIFFITHS ET AL. --- | 1-6 | C07D207/16 C07D207/14 C07D407/06 A61K31/40 |
| Y | CHEMICAL ABSTRACTS, vol. 120, no. 21, 23 May 1994 Columbus, Ohio, US; abstract no. 271177g, * abstract * & JP-A-05 213 957 (TSUMURA AND CO.) --- | 1-6 | |
| Y | CHEMICAL ABSTRACTS, vol. 120, no. 5, 31 January 1994 Columbus, Ohio, US; abstract no. 46429w, * abstract * & NAUNYN-SCHMIEDEBERG'S ARCH. PHARMACOL., vol. 348, no. 5, 1993 pages 478-485, P.C. WALDMEIER ET AL. --- | 1-6 | TECHNICAL FIELDS SEARCHED (Int.Cl.6): C07D A61K |
| Y | CHEMICAL ABSTRACTS, vol. 119, no. 3, 19 July 1993 Columbus, Ohio, US; abstract no. 21035f, * abstract * & BIOCHEM. SOC. TRANS., vol. 21, no. 2, 1993 page 111s J. DUNLOP ET AL. --- -/-- | 1-6 | |

The present search report has been drawn up for all claims

| Place of search | Date of completion of the search | Examiner |
|---|---|---|
| BERLIN | 15 December 1995 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 95 30 5800

| Category | Citation of document with indication, where appropriate, of relevant passages | Relevant to claim | CLASSIFICATION OF THE APPLICATION (Int.Cl.6) |
|---|---|---|---|
| Y | CHEMICAL ABSTRACTS, vol. 114, no. 9, 4 March 1991 Columbus, Ohio, US; abstract no. 82454k, * abstract * & JOURNAL OF MEDICINAL CHEMISTRY, vol. 34, no. 2, 1991 WASHINGTON US, pages 717-725, R.J. BRIDGES ET AL. --- | 1-6 | |
| Y | CHEMICAL ABSTRACTS, vol. 119, no. 5, 2 August 1993 Columbus, Ohio, US; abstract no. 41525u, * abstract * & NEUROSCI. LETT., vol. 153, no. 1, 1993 pages 107-110, J. CARTMELL ET AL. --- | 1-6 | |
| Y | CHEMICAL ABSTRACTS, vol. 117, no. 13, 28 September 1992 Columbus, Ohio, US; abstract no. 125166z, * abstract * & J. NEUROCHEM., vol. 59, no. 2, 1992 pages 610-615, S.P.H. ALEXANDER ET AL. --- -/-- | 1-6 | TECHNICAL FIELDS SEARCHED (Int.Cl.6) |

The present search report has been drawn up for all claims

| Place of search | Date of completion of the search | Examiner |
|---|---|---|
| BERLIN | 15 December 1995 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 95 30 5800

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | CHEMICAL ABSTRACTS, vol. 115, no. 17, 28 October 1991 Columbus, Ohio, US; abstract no. 174551p, * abstract * & EUR. J. PHARMACOL., MOL. PHARMACOL. SECT., vol. 207, no. 4, 1991 pages 351-353, D.D. SCHOEPP ET AL. --- | 1-6 | |
| Y | CHEMICAL ABSTRACTS, vol. 112, no. 5, 29 January 1990 Columbus, Ohio, US; abstract no. 30730m, * abstract * & JOURNAL OF MEDICINAL CHEMISTRY, vol. 33, no. 1, 1990 WASHINGTON US, pages 374-380, U. MADSEN ET AL. --- | 1-6 | |
| Y | CHEMICAL ABSTRACTS, vol. 98, no. 7, 14 February 1983 Columbus, Ohio, US; abstract no. 47105b, * abstract * & NEUROCHEM. RES., vol. 7, no. 9, 1982 pages 1119-1133, J. DAVIES ET AL. ----- | 1-6 | TECHNICAL FIELDS SEARCHED (Int.Cl.6) |

The present search report has been drawn up for all claims

| Place of search | Date of completion of the search | Examiner |
|---|---|---|
| BERLIN | 15 December 1995 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)